(19) Europäisches Patentamt
European Patent Office
Office européen des brevets

(11) EP 4 534 003 A1

(12) EUROPEAN PATENT APPLICATION
published in accordance with Art. 153(4) EPC

(43) Date of publication:
**09.04.2025 Bulletin 2025/15**

(21) Application number: **23815658.2**

(22) Date of filing: **28.04.2023**

(51) International Patent Classification (IPC):
***A61B 5/01*** *(2006.01)* ***C09K 9/02*** *(2006.01)*

(52) Cooperative Patent Classification (CPC):
**A61B 5/01; C09K 9/02**

(86) International application number:
**PCT/JP2023/016835**

(87) International publication number:
**WO 2023/233897 (07.12.2023 Gazette 2023/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.05.2022 JP 2022088104**

(71) Applicant: **Keio University**
**Tokyo, 108-8345 (JP)**

(72) Inventors:
- **OAKI, Yuya**
  **Yokohama-shi, Kanagawa 223-8522 (JP)**
- **EDAGAWA, Aya**
  **Yokohama-shi, Kanagawa 223-8522 (JP)**
- **MATSUDA, Satoru**
  **Tokyo 160-8582 (JP)**
- **KAWAKUBO, Hirofumi**
  **Tokyo 160-8582 (JP)**
- **SHIBATA, Risako**
  **Yokohama-shi, Kanagawa 223-8522 (JP)**

(74) Representative: **Winter, Brandl - Partnerschaft mbB**
**Alois-Steinecker-Straße 22**
**85354 Freising (DE)**

# (54) TEMPERATURE IMAGING DEVICE AND METHOD FOR MANUFACTURING SAME

(57) Provided is a temperature imaging device that can visualize a temperature change by color even with a large area. The temperature imaging device includes: a base material; and a composite having polyallylamine introduced between layers of poly-10,12-pentacosadiynoic acid, the composite being incorporated into the base material or caused to adhere onto the base material.

Fig. 3

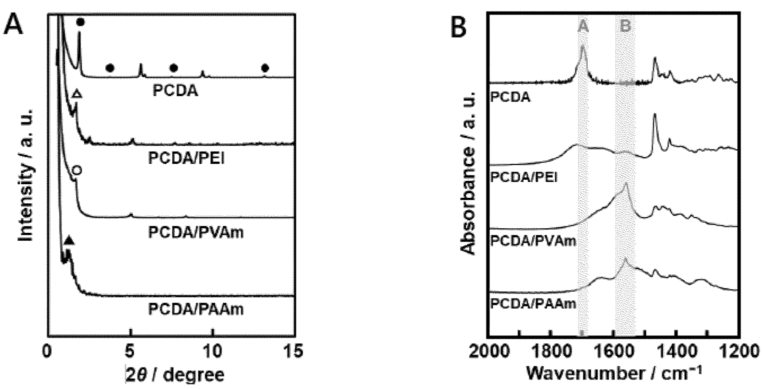




Processed by Luminess, 75001 PARIS (FR)

## Description

Technical Field

The present invention relates to a temperature imaging device and a method of producing the device.

Background Art

Surgeons perform practices while being concerned about thermal damage to surrounding organs when using an electric scalpel. Temperature imaging may be performed by infrared thermography, but as more accuracy is required in temperature measurement and imaging, a more expensive apparatus is required. In addition, it is not easy to always prepare such apparatus in medical settings where an electric scalpel is used.

As a temperature indicator patch having a relatively simple configuration, there is a disclosure of a temperature indicator patch containing a transparent resin and a thermochromic pigment, in which the color development temperature of the thermochromic pigment is from 80°C to 110°C (Patent Literature 1).

The inventors of the present invention have conducted a research on layered polydiacetylene (PDA) exhibiting a color change in response to a temperature. PDA is a polymer exhibiting a color change from blue to red in response to an external stimulus. It has been known that temperature responsiveness can be adjusted by intercalating various chemical species (guests) between layers of a layered structure of 10,12-pentacosadiynoic acid (PCDA), which is a kind of PDA. It has been confirmed that a temperature-responsive color change is caused in a color different from a color change of PDA by, for example, intercalating a metal ion (Non-patent Literature 1) between PDA layers. In addition, it has been confirmed that a temperature range that shows reversibility of the color change is widened in accordance with an increase in number of carbon atoms of an intercalated amine by intercalating an alkylamine (Non-patent Literature 2) or a dialkylamine (Non-patent Literature 3) between PDA layers.

In recent years, the inventors of the present invention have found that, when polyallylamine is composited between the layers, the color gradually changes in accordance with the temperature between about room temperature to 100°C, and hence temperature imaging in a wide temperature range is enabled (Non-patent Literature 4). However, the guest composited sample has been a powder sample, or it has been difficult to produce a temperature imaging device having a large area according to a related-art coating method for a base material.

Citation List

Patent Literature

PTL 1: JP 6801878 B2

Non-patent Literature

NPL 1: M. Okaniwa, et al. Adv. Funct. Mater., 2016, 26, 3463-3471

NPL 2: M. Takeuchi, et al. ACS Appl. Mater. Interfaces, 2017, 9, 16546-16552

NPL 3: Y. Oaki, Y. Ishijima, H. Imai, Polymer Journal, 2018, 50, 319-326

NPL 4: Aya Edagawa, Hiroaki Imai, and Yuya Oaki, The 70th SPSJ Annual Meeting "Reduction in Temperature and Increase in Width of Temperature-responsive Color Change by Flexibility Control of Layered Polydiacetylene"


Summary of Invention

Technical Problem

An object of the present invention is to provide a temperature imaging device including a composite in a base material or on the base material, the composite having polyallylamine introduced between layers of polydiacetylene, and a method of producing the device, each of which can achieve even a large-capacity device.

Solution to Problem

The present invention includes embodiments described below.

Item 1.

A temperature imaging device including: a base material; and a composite having polyallylamine introduced between layers of poly-10,12-pentacosadiynoic acid, the composite being incorporated into the base material or caused to adhere onto the base material.

Item 2.

The temperature imaging device according to Item 1, wherein the temperature imaging device has an initial value of a red intensity "x" before heating of 0.280 or more and a value of a change amount Δx of the red intensity "x" when a color change is caused by heating to 100°C of 0.025 or more.

Item 3.

A temperature imaging device including: a base material; and a composite of poly-10,12-pentacosadiynoic acid, polyallylamine, and an amine compound, the composite being incorporated into the base material or caused to adhere onto the base material, wherein at least part of the polyallylamine and/or the amine compound is introduced between layers of the poly-10,12-pentacosadiynoic acid.

Item 4.

The temperature imaging device according to Item 1 or 3, wherein a plurality of droplets of the composite adhere to the base material in a substantially spherical shape under a dispersed state.

Item 5.

The temperature imaging device according to Item 1 or 3, wherein the base material has a sheet shape, and wherein the temperature imaging device is a sheet-shaped device.

Item 6.

The temperature imaging device according to Item 1 or 3, wherein the base material includes a woven fabric or a non-woven fabric.

Item 7.

The temperature imaging device according to Item 1, wherein the base material is a sheet-shaped base material having a length of 100 mm or more and a width of 100 mm or more.

Item 8.

The temperature imaging device according to Item 1, wherein the temperature imaging device has a color change start temperature lower than 30°C and a color change end temperature higher than 80°C.

Item 9.

A method of estimating a specific heat of a liquid, the method including:

heating a container containing the liquid, the container having been sealed, to a certain temperature;
measuring a color change of the temperature imaging device of Item 1 or 3 attached to the container;
calculating a specific heat equivalent value of the liquid from a time period from a start of heating until the color change stops and a change amount Δx of a red intensity "x" of the temperature imaging device from the start of the heating until the color change stops; and
substituting the specific heat equivalent value of the liquid into a functional formula for a specific heat and a specific heat equivalent value to calculate the specific heat of the liquid.

Item 10.

A method of examining a temperature change of a test site, the method including:

arranging the temperature imaging device of Item 1 or 3 on the test site; and
detecting a color change of the temperature imaging device arranged on the test site after heating.

Item 11.

The method according to Item 10, wherein the detecting the color change includes detecting, after heating with an energy device for a surgery, the color change of the temperature imaging device arranged on an affected part subjected to heating treatment or a vicinity thereof.

Item 12.

An energy device for a surgery including a coating caused to adhere to the energy device, the coating including a composite having polyallylamine introduced between layers of poly-10,12-pentacosadiynoic acid.

Item 13.

An energy device for a surgery including a coating caused to adhere to the energy device, the coating including a composite containing poly-10,12-pentacosadiynoic acid, polyallylamine, and an amine compound, the composite having at least part of the polyallylamine and/or the amine compound introduced between layers of the poly-10,12-pentacosadiynoic acid.

Item 14.

A method of producing a temperature imaging device, the method including:

applying, to a base material, a composition containing 10,12-pentacosadiynoic acid, polyallylamine having a unit represented by the following general formula (1), and a solvent by spray coating;
removing the solvent; and
polymerizing the 10,12-pentacosadiynoic acid to form a composite having the polyallylamine introduced between layers of poly-10,12-pentacosadiynoic acid:

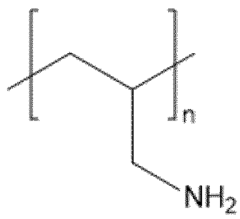

(1)

where "n" represents an integer.

Item 15.

A method of producing a temperature imaging device, the method including:

applying, to a base material, a composition containing 10,12-pentacosadiynoic acid, polyallylamine having a unit represented by the following general formula (1), an amine compound, and a solvent by spray coating;
removing the solvent; and
polymerizing the 10,12-pentacosadiynoic acid to form a composite having at least part of the polyallylamine and/or the amine compound introduced between layers of poly-10,12-pentacosadiynoic acid:

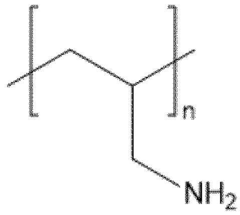

(1)

where "n" represents an integer.

Item 16.

The method according to Item 14 or 15, wherein the forming the composite includes forming the composite so that the composite adheres to the base material in a substantially spherical shape.

Advantageous Effects of Invention

According to the temperature imaging device of the present invention, temperature imaging in a wide temperature range can be performed. In addition, according to the method of producing a temperature imaging device of the present invention, the temperature imaging device with which temperature imaging in a wide temperature range can be performed can be produced.

Brief Description of Drawings

Figs. 1 show the formation of a layered polymer with 10,12-pentacosadiynoic acid. Fig. 1(A) shows 10,12-pentacosadiynoic acid (PCDA). Fig. 1(B) shows the formation of a lamellar structure with PCDA. Fig. 1(C) shows the formation of a polymer PDA by topochemical polymerization. Fig. 1(D) shows the formation of a composite having a polymer guest introduced between layers of PDA.

Fig. 2 is a schematic view of a temperature imaging device according to one embodiment.

Fig. 3(A) shows XRD patterns of a PCDA crystal and PCDA/alkyl-based polymer guests PEI, PVAm, and PAAm. Fig. 3(B) shows IR spectra of the PCDA crystal and the PCDA/alkyl-based polymer guests.

Fig. 4(A) shows XRD patterns of the PCDA crystal and PCDA/pyridine-based polymer guests PVP2 and PVP4. Fig. 4(B) shows IR spectra of the PCDA crystal and the PCDA/pyridine-based polymer guests.

Figs. 5 show photographs of PCDA and the PCDA/polymer guests after UV irradiation. Fig. 5(A) shows PDA (polymerized PCDA). Fig. 5(B) shows PDA/PEI. Fig. 5(C) shows PDA/PVAm. Fig. 5(D) shows PDA/PAAm. Fig. 5(E) shows PDA/PVP4. Fig. 5(F) shows PDA/PVP2.

Fig. 6 shows changes in peak intensity ratios of the respective composites with respect to UV irradiation time, that is, polymerization rates.

Fig. 7 shows color change photographs of PDA and PDA/alkyl-based polymer guests.

Figs. 8 show image analysis results of the color change photographs of PDA and the PDA/alkyl-based polymer guests. Fig. 8(A) is a chromaticity diagram. Fig. 8(B) shows temperature-dependent changes in distances between a measurement starting point and respective plots.

Figs. 9 show UV-vis spectra. Fig. 9(A) shows PDA. Fig. 9(B) shows PDA/PAAm.

Fig. 10(A) shows a torsion angle of a conjugated chain of PDA. Fig. 10(B) shows a torsion angle of a conjugated chain of PDA/PAAm.

Fig. 11 shows DSC measurement results of PDA and the PDA/alkyl-based polymer guests.

Figs. 12 show optical microscope images of samples each produced by a LbL method. Fig. 12(A) shows PDA/PEI. Fig. 12(B) shows PDA/PVAm. Fig. 12(C) shows PDA/PAAm.

Figs. 13 show SEM images of the samples each produced by the LbL method. Fig. 13(A) shows PDA/PEI. Fig. 13(B) shows PDA/PVAm. Fig. 13(C) shows PDA/PAAm.

Figs. 14 show comparison among silk cloths coated by various coating methods. Fig. 14(A) is a photograph taken with a camera for a layer-by-layer method (LbL method). Fig. 14(B) is a photograph taken with the camera for a direct synthesis method. Fig. 14(C) is a photograph taken with the camera for a spray coating method. Fig. 14(D) is an optical micrograph for the LbL method. Fig. 14(E) is an optical micrograph for the direct synthesis method. Fig. 14(F) is an optical micrograph for the spray coating method.

Figs. 15 are photographs of application of PDA and PDA/polymer guests to a PMMA substrate by spray coating. Fig.

15(A) shows PDA. Fig. 15(B) shows PDA/PEI. Fig. 15(C) shows PDA/PVAm. Fig. 15(D) shows PDA/PAAm.

Figs. 16 are SEM images of application of PDA and the PDA/polymer guests to the PMMA substrate by spray coating. Fig. 16(A) shows PDA. Fig. 16(B) shows PDA/PEI. Fig. 16(C) shows PDA/PVAm. Fig. 16(D) shows PDA/PAAm.

Fig. 17 shows a relationship between a specific heat equivalent value and a specific heat of a literature value. (a) represents silicone oil, (b) represents ethanol, (c) represents water, and (d) represents a 50 wt% ethanol aqueous solution.

Fig. 18 is a photograph of a temperature imaging sheet attached to a piece of pork in a state before being subjected to heat treatment with an electric scalpel.

Fig. 19(A) is a photograph of a silk cloth coated with PDA/PAAm arranged on a piece of meat, and then cut with an electric scalpel A. Fig. 19(B) is a photograph of the silk cloth coated with PDA/PAAm arranged on the piece of meat, and then cut with an electric scalpel B. Fig. 19(C) is a graph showing relationships between a distance from a cut site of the piece of meat (horizontal axis), and a red intensity ("x", left axis) and a temperature (right axis) of a device when the electric scalpels A and B are used.

Figs. 20 each show color change behavior quantitative analysis of PAAm/PDA-guest composites based on image analysis. A vertical axis represents a red intensity change Δx determined by the image analysis. Fig. 20(A) shows color changes of composites containing PAAm in the respective introduction amounts at the time of temperature increase. Fig. 20(B) shows color changes of the composites containing PAAm in the respective introduction amounts at the time of cooling.

Fig. 21 shows color change behavior quantitative analysis of the PAAm/PDA-guest composites based on the image analysis. A vertical axis represents the red intensity change Δx determined by the image analysis. Solid line: color changes of composites containing pXy in three kinds of amounts at the time of temperature increase, dotted line: color changes of the composites containing pXy in three kinds of amounts at the time of cooling.

Fig. 22 shows FT-IR measurement results of samples I to V of the composites.

Figs. 23 show RGB image analysis results of a sample II(A), a sample III(B), and a sample IV(C) of the composite.

Figs. 24 are SEM images. Fig. 24(A) is a secondary electron image of PDA-p-Xy. Fig. 24(B) is a secondary electron image of PAAm/PDA-p-Xy. Fig. 24(C) is a backscattered electron image of PDA-p-Xy. Fig. 24(D) is a backscattered electron image of PAAm/PDA-p-Xy.

Fig. 25 is a schematic view of a phase separation structure of PAAm and PDA-p-Xy.

Figs. 26 show PAAm/PCDA-guests and PCDA-guests. Fig. 26(A) shows IR measurement results. Fig. 26(B) shows XRD measurement results.

Fig. 27 is an optical micrograph of color change behaviors of PAAm/PDA-guests and PDA-guests.

Fig. 28 shows PAAm/PDA-p-Xy-PAAm coating of a filter paper substrate and a silk cloth substrate using three kinds of coating methods.

Figs. 29 are optical images of substrates before coating (left) and the substrates after PAAm/PDA-p-Xy coating and polymerization (right). Fig. 29(A) shows polyurethane. Fig. 29(B) shows hard polyethylene. Fig. 29(C) shows polypropylene. Fig. 29(D) shows a silk cloth. Fig. 29(E) shows a polyethylene fiber. Fig. 29(F) shows polystyrene.

Fig. 30 shows a color change behavior of a PAAm/PDA-p-Xy silk cloth substrate.

Fig. 31 shows an image analysis result of the color change behavior of the PAAm/PDA-p-Xy silk cloth substrate.

## Description of Embodiments

A first embodiment of the present invention is described in detail.

10,12-Pentacosadiynoic acid (PCDA, represented by reference numeral 1), which is a kind of diacetylene (DA), is an amphipathic molecule having a carboxy group at its terminal (Fig. 1(A)), and forms a lamellar structure through self-assembly (Fig. 1(B)). PCDA is aligned with a molecular length of 3.18 nm and a molecular inclination of from about 45° to about 50°, and hence has an interlayer spacing of 4.61 nm. Topochemical polymerization progresses through UV irradiation and heating (Fig. 1(C)) to present a blue color. PDA obtained by polymerization of PCDA exhibits an irreversible color change from blue to red when heated to about 65°C. The polymerized PCDA is hereinafter sometimes referred to as "PDA".

It has been known that a temperature-responsive color change can be controlled by forming composites having various guests introduced between layers of PCDA. When interlayer guests each have a low molecular weight, the guests are aligned between the layers of PCDA without any gap through van der Waals' forces, and hence an alignment property and the ease of polymerization increase, though the flexibility of a layer structure is low. When the interlayer guests each have a high molecular weight, crystallinity decreases because of the mobility of a polymer chain, and hence the alignment property and the ease of polymerization decrease, though the flexibility of the layer structure is high.

In this research, a success was made in extending the control range of heat stimulus responsiveness by an increase in flexibility of the layer structure by producing a composite having a specific polymer guest (Fig. 1(D), represented by reference numeral 2) introduced between the layers of PCDA and producing a polymer thereof.

Specifically, for example, a polymerization degree or a color change in the case where each of the following five kinds of polymer guests (I) to (V) was used as an interlayer guest for the PCDA layers was observed. As a result, it was found that the polymerization rate was low in a composite containing a pyridine-based guest, that is, poly(4-vinylpyridine) (IV) or poly(2-vinylpyridine) (V), and the polymerization sufficiently progressed in a composite containing an alkyl-based guest, that is, polyethyleneimine (I), polyvinylamine (II), or polyallylamine (III). In addition, it was found that, in each composite containing an alkyl-based guest between layers of poly-PCDA (PDA), a color change start temperature was reduced and a color change temperature width was increased as compared to those of a PCDA polymer (PDA) free of any guest, and that the color change temperature was further reduced and further increased in width as the guest polymer becomes more bulky in the order of the polyethyleneimine (I), the polyvinylamine (II), and the polyallylamine (III). In particular, a composite of poly-PCDA (PDA) and the polyallylamine (III) had a color change start temperature of -50°C, a color change end temperature of 220°C, and a color change temperature width of 270°C, and hence a color change was so clear as to be observable with the naked eye in a practical temperature range of from 0°C to 100°C.

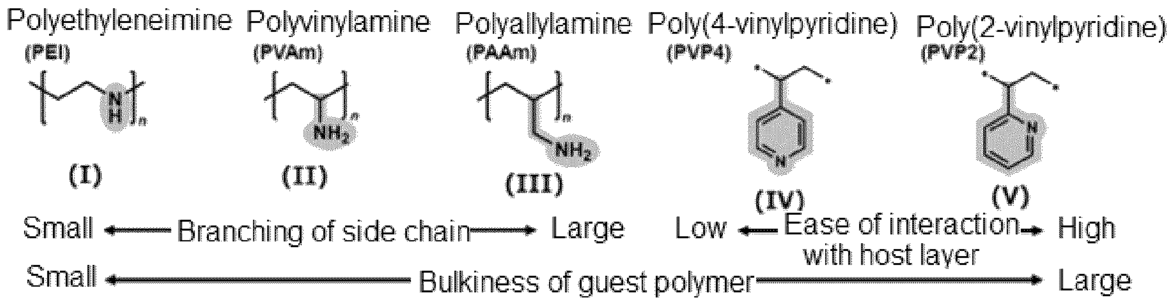


More surprisingly, in order to increase the area of the temperature imaging device of a composite having polyallylamine introduced between the layers of poly-PCDA (PDA), a PCDA composite produced by using each of the above-mentioned five kinds of polymer guests (I) to (V) was applied to a large-area substrate, in which the two-dimensional (length×width) dimensions of a surface to which the composite was applied were each 1 cm or more, by spray coating. As a result, uniform coating was achieved with only the composite containing the polyallylamine (III).

Accordingly, in the temperature imaging device of the first embodiment of the present invention, the polyallylamine (III) is used as the guest polymer.

The temperature imaging device of the first embodiment of the present invention includes: a base material; and a composite having polyallylamine introduced between layers of poly-10,12-pentacosadiynoic acid, the composite being incorporated into the base material or caused to adhere onto the base material. An example of a temperature imaging device 10 including a base material 11 having a flat plate shape and a composite 12 caused to adhere onto the base material 11 is illustrated in Fig. 2.

The polyallylamine is polyallylamine having a unit represented by the following general formula (1):

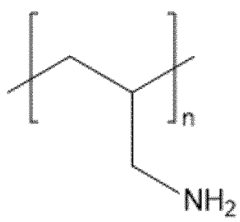

(1)

where "n" represents an integer.

The number-average molecular weight of the polyallylamine having the unit represented by the general formula (1) is preferably from 10,000 to 20,000.

In some embodiments, in the temperature imaging device, the particle diameter of the composite may be observed with, for example, an electron microscope, and the composite may have a diameter of, for example, from 1 μm to 10 μm. The average particle diameter of the composite having a substantially spherical shape may also be measured, and composites each having a diameter of 1 μm or more observed with, for example, an electron microscope are randomly selected and the diameters of 20 or more composites are measured. An average particle diameter thereof may be from 1 μm to 10 μm.

The kind of the base material is not particularly limited, and examples thereof include: synthetic resins, such as a thermoplastic resin and a thermosetting resin; elastomers, such as a thermoplastic elastomer and a thermosetting elastomer; rubbers; metals; paper; and fabrics of a natural material and a synthetic material. The fabric includes a woven

fabric and a non-woven fabric. Examples of the natural material include, but not limited to, cotton and silk. Examples of the synthetic material include, but not limited to, synthetic resins, such as polyester, polyolefin, and polyurethane.

In some embodiments, the base material is preferably a woven fabric or a non-woven fabric from the viewpoint of the ease of adhesion of the composite having the polyallylamine introduced between the layers of polydiacetylene to the base material. A particle size of the composite of the embodiment of the present invention is reduced as compared to that of a composite using a guest polymer except the polyallylamine, and hence a small particle easily adheres to a fiber material for the woven fabric or the non-woven fabric. Thus, uniform coating can be achieved. Accordingly, the composite is also suitable for large-area coating.

The shape of the base material is not particularly limited, but is, for example, a sheet shape. The base material may be a flat plate-shaped sheet, or may be a sheet having a curved surface.

The dimensions of the base material are not particularly limited, but the two-dimensional dimensions of the surface of the base material to which the composite having the polyallylamine introduced between the layers of the polydiacetylene is applied are preferably 1 mm to 100 cm×1 mm to 100 cm square, more preferably 1 cm to 100 cm×1 cm to 100 cm square, and when the base material has a large area, the dimensions are 10 cm to 100 cm×10 cm to 100 cm square. A ratio between the respective two-dimensional dimensions is preferably from 100:1 to 1:100, more preferably from 10:1 to 1:10.

When the base material is a sheet base material, the dimensions of the base material are not particularly limited, but are, for example, a length of from 1 mm to 100 cm and a width of from 1 mm to 100 cm, preferably a length of from 1 cm to 100 cm and a width of from 1 cm to 100 cm, and when the base material has a large area, the dimensions are a length of from 10 cm to 100 cm and a width of from 10 cm to 100 cm. The ratio "length:width" is preferably from 100:1 to 1:100, more preferably from 10:1 to 1:10. The thickness is not particularly limited, but is preferably smaller than the dimensions of the length and the width.

When the temperature imaging device is a temperature imaging sheet having a large area, the two-dimensional dimensions of the length and width of each of the base material and the temperature imaging sheet are each preferably more than 10 cm.

In the temperature imaging device, part or the whole of the base material may be surrounded by a transparent film as long as heat transfer to the composite having the polyallylamine introduced between the layers of poly-10,12-pentacosadiynoic acid is not prevented. The thickness of the film is not particularly limited, and is preferably 200 μm or less. When the base material is surrounded by such film, a waterproof property and/or an antifouling property can be imparted to the base material while the observation of the color change of the temperature imaging device is enabled.

The temperature imaging device of the first embodiment of the present invention has a wide color change temperature range when increased in temperature with a heating apparatus. The color change of the temperature imaging device of the first embodiment of the present invention may be directly observed with the naked eye, or may be photographed to form an image and provided as numerical data.

The color change start temperature of the temperature imaging device of the first embodiment of the present invention is preferably lower than 40°C, lower than 30°C, lower than 20°C, lower than 10°C, or lower than 0°C. The color change start temperature of the temperature imaging device of the first embodiment of the present invention is more preferably lower than 30°C. When the color change is started within such temperature ranges, a color change can be detected at a temperature lower than room temperature or a body temperature. In addition, the color change end temperature of the temperature imaging device of the first embodiment of the present invention is preferably higher than 60°C, higher than 70°C, higher than 80°C, higher than 90°C, or higher than 100°C. When the color change is ended within such temperature ranges, a color change along with a temperature increase to a practical temperature, such as a temperature at which a protein is denatured or a temperature lower than the boiling point of water, can be detected.

In some embodiments, the color change start temperature of the temperature imaging device is lower than 30°C, and the color change end temperature thereof is higher than 80°C. In such temperature ranges, a color change at a temperature including a temperature of daily life, a body temperature of a subject, and a denaturation temperature of a protein can be detected.

In some embodiments, a color change occurs over the entire range of from at least 30°C to 80°C. In such temperature ranges, a color change caused by a heat stimulus can be detected in a wide range including a temperature of daily life, a body temperature of a subject, and a denaturation temperature of a protein. In some embodiments, a color change occurs over the entire range of from at least 20°C to 100°C. In some embodiments, a color change occurs over the entire range of from at least 0°C to 100°C.

In the temperature imaging device of the first embodiment of the present invention, when the color change of the temperature imaging device between before heating and after heating to 100°C through an increase in temperature is subjected to image analysis, an initial value of a red intensity "x" before heating is represented by $x_0$, a red intensity after heating to 100°C is represented by $x_1$, and a change amount of the red intensity caused by heating is represented by $\Delta x = x_1 - x_0$, the initial value $x_0$ of the red intensity "x" before heating is preferably 0.280 or more, and a value of the change amount $\Delta x$ of the red intensity "x" when a color change is caused by heating to 100°C is preferably 0.025 or more. The

temperature at the time of the measurement of the initial value $x_0$ of the red intensity "x" before heating is preferably 20°C. The red intensity "x" may be calculated from x=X/(X+Y+Z) by calculating R, G, and B values from an image of the temperature imaging device with software (ImageJ), and converting the values to an XYZ color system through use of Rec.709 standard. When the value of the change amount $\Delta x$ of the red intensity "x" is 0.025 or more, the color density of the temperature imaging device is easily viewed, and the change amount $\Delta x$ of the red intensity "x" when a color change is caused by heating to 100°C is large. Accordingly, such temperature imaging device is suitable for the detection of a color change caused by heating.

The temperature imaging device of the first embodiment of the present invention may be used for various applications in each of which detection of a temperature change is required. For example, an energy device for a surgery such as an electric scalpel to be used at the time of a surgery may cause thermal damage around an affected part requiring treatment, but the temperature imaging device of the first embodiment of the present invention can be directly mounted near the affected part, and hence the temperature near the affected part and a temperature change thereof by the treatment can be visualized by color. The temperature imaging device of the first embodiment of the present invention is particularly useful because a color change can be made from near a body temperature to about 80°C. Thus, a temperature distribution around the energy device for a surgery can be visualized and quantified, and hence what operation or usage history may cause a risk can be recognized at a glance, leading to usage and development of a secure and safe surgery or a secure and safe medical instrument.

The temperature imaging device of the first embodiment of the present invention may be used for, for example, examining or detecting a temperature change of a test site of a subject or a vicinity thereof. The subject may be a living organism such as a human or may be a non-living matter.

In some embodiments, the method of examining a temperature change of a test site, the method includes: arranging the temperature imaging device of the first embodiment of the present invention on the test site; and detecting a color change of the temperature imaging device arranged on the test site after heating. The term "test site" refers to a site subjected to heating treatment or a vicinity thereof. The term "vicinity of the site subjected to heating treatment" refers to, for example, a region from the site subjected to heating treatment to a position distant therefrom by 5 cm, preferably refers to a region from the site subjected to heating treatment to a position distant therefrom by 3 cm, and more preferably refers to a region from the site subjected to heating treatment to a position distant therefrom by 1 cm. The vicinity of the site subjected to heating treatment may be a position adjacent to the site subjected to heating treatment. The temperature imaging device is particularly preferably the temperature imaging sheet.

In some embodiments, the method of examining a temperature change of a test site is a method of examining a temperature change of the vicinity of the site subjected to heating treatment (e.g., an affected part), the method including: arranging the above-mentioned temperature imaging device of the first embodiment of the present invention on the vicinity of a certain site; and detecting a color change of the temperature imaging device arranged on the vicinity of the site after subjecting the site to heating treatment. The temperature imaging device is particularly preferably the temperature imaging sheet.

Examples of the heating apparatus include: an energy device for a surgery such as an electric scalpel; and a heater. The term "vicinity of the site subjected to heating treatment" refers to, for example, a region from the site subjected to heating treatment to a position distant therefrom by 5 cm, preferably refers to a region from the site subjected to heating treatment to a position distant therefrom by 3 cm, and more preferably refers to a region from the site subjected to heating treatment to a position distant therefrom by 1 cm. The vicinity of the site subjected to heating treatment may be a position adjacent to the site subjected to heating treatment.

In some embodiments, the temperature imaging sheet may be cut to a required size when required and used on site. For example, when a doctor handles an electric scalpel, how far heat is conducted around a site where the electric scalpel is used or how much heat a main body of the electric scalpel has through its repeated use can be viewed in real time by a color change of the temperature imaging sheet of the first embodiment of the present invention. Accordingly, a risk of thermal damage to the site subjected to heating treatment with the electric scalpel or to surrounding organs or nerves can be visually understood. The temperature imaging sheet is useful also for a medical equipment manufacturer in that performance or safety of the heating apparatus such as the electric scalpel can be visually observed.

In some embodiments, there is provided an energy device for a surgery including a coating caused to adhere to the energy device, the coating including a composite having polyallylamine introduced between layers of poly-10,12-pentacosadiynoic acid. The term "energy device for a surgery" refers to a device having a function of incision, coagulation, and/or hemostasis of a biological target portion by releasing energy from a microwave, a high-frequency wave, or an ultrasonic wave to be allowed to act on the biological target portion. The energy device is suitably used for a surgery. Examples of the energy device for a surgery include a scalpel, thumb forceps, forceps, and a snare. For example, by incorporating the coating including the composite into the main body of the electric scalpel as a temperature indicator, it can be expected that a surgery can be performed while the temperature at the time of use is visually monitored.

As described above, each of the temperature imaging device of the first embodiment of the present invention, and the heating apparatus including the coating caused to adhere to the heating apparatus, the coating including the

composite having polyallylamine introduced between the layers of poly-10,12-pentacosadiynoic acid may be able to comprehensively solve the problem of thermal damage at the time of the use of the heating apparatus.

Next, a method of producing a temperature imaging device of the first embodiment of the present invention is described.

The method of producing a temperature imaging device of the first embodiment of the present invention includes:

applying, to a base material, a solution containing 10,12-pentacosadiynoic acid, polyallylamine having a unit represented by the following general formula (1), and a solvent by spray coating;
removing the solvent; and
polymerizing the 10,12-pentacosadiynoic acid to form a composite having the polyallylamine introduced between layers of poly-10,12-pentacosadiynoic acid:

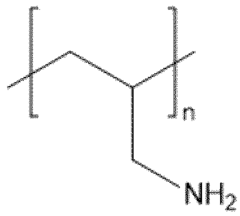 (1)

where "n" represents an integer.

The number-average molecular weight of the polyallylamine having a unit represented by the general formula (1) is preferably from 10,000 to 20,000.

The solvent of the solution to be used for the spray coating is not particularly limited, but is preferably an organic solvent. Examples thereof may include hexane, ethanol, and a mixed solvent thereof.

A ratio between 10,12-pentacosadiynoic acid and the polyallylamine having a unit represented by the following general formula (1) in the solution is not particularly limited as long as a composite having polyallylamine introduced between the layers of poly-10,12-pentacosadiynoic acid can be formed, but a molar ratio between a carboxy group of 10,12-pentacosadiynoic acid and an amino group of polyallylamine is preferably set to 1:2.

Before a solution containing 10,12-pentacosadiynoic acid, polyallylamine having a unit represented by the following general formula (1), and a solvent is applied to a base material by spray coating, the solution is preferably dispersed by an ultrasonic wave in order to miniaturize and uniformize particles of a solute.

The application of the solution to the base material is performed by applying the solution to the base material in an upright state by spray coating from a position distant therefrom. A distance between the base material and an ejection orifice of a container containing the solution may be appropriately selected.

The kind and shape of the base material are as described above.

The removal of the solvent may be performed by natural drying, or may be performed by forced drying by a known drying technology.

The polymerization of 10,12-pentacosadiynoic acid may be performed by UV irradiation. There is known topochemical polymerization of 10,12-pentacosadiynoic acid by UV irradiation.

In some embodiments, the forming the composite includes forming the composite so that the composite adheres to the base material in a substantially spherical shape. The particle diameter of the composite having a substantially spherical shape may be observed with, for example, an electron microscope, and the composite may have a diameter of, for example, from 1 μm to 10 μm. The average particle diameter of the composite having a substantially spherical shape may also be measured, and composites each having a diameter of 1 μm or more observed with, for example, an electron microscope are randomly selected and the diameters of 20 or more composites are measured. An average particle diameter thereof may be from 1 μm to 10 μm. A person skilled in the art who has read this description can adjust the particle diameter of the composite having a substantially spherical shape adhering to the base material so as to satisfy a desired range by, for example, the selection of the conditions of the ultrasonic treatment.

According to the method of producing a temperature imaging device of the first embodiment of the present invention by the spray coating, a desired substrate can be simply and uniformly coated, and hence a large-area device can be produced.

Next, a second embodiment of the present invention is described in detail. In the second embodiment of the present invention, differences from the first embodiment are mainly described.

In this description, the term "low-molecular-weight amine" refers to an amine compound having a molecular weight of 600 or less, and preferably refers to an amine compound having a molecular weight of 300 or less. The molecular weight of the low-molecular-weight amine may be determined from a molecular formula when the molecular formula can

be specified. When the molecular formula cannot be specified, a number-average molecular weight (in terms of polystyrene) measured by gel permeation chromatography (GPC) is used.

A temperature imaging device of the second embodiment of the present invention includes: a base material; and a composite of poly-10,12-pentacosadiynoic acid, polyallylamine, and a low-molecular-weight amine, the composite being incorporated into the base material or caused to adhere onto the base material, wherein at least part of the polyallylamine and/or the amine compound is introduced between layers of poly-10,12-pentacosadiynoic acid.

In the temperature imaging device of the second embodiment of the present invention, polyallylamine and/or an amine compound is used as a guest molecule. The molecular weight of the amine compound may be calculated from a molecular formula. The amine compound is free of polyallylamine.

The polyallylamine is polyallylamine having the unit represented by the general formula (1) described above:

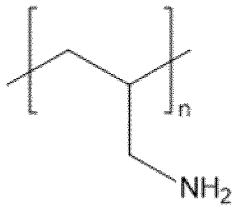

(1)

where "n" represents an integer.

The number-average molecular weight of the polyallylamine having the unit represented by the general formula (1) is preferably from 10,000 to 20,000.

The low-molecular-weight amine may be a monoamine, a diamine, or a triamine. Examples of the low-molecular-weight amine include, but not limited to, an aromatic amine (monoamine or diamine), an aliphatic amine (monoamine or diamine), an alicyclic amine (monoamine or diamine), and an amino acid.

The blending ratio of PCDA, PAAm, and the low-molecular-weight amine in the composite is preferably 0.1 to 0.9 of PAAm in terms of molar ratio and 0.5 to 5 of the low-molecular-weight amine in terms of molar ratio, more preferably 0.3 to 0.7 of PAAm in terms of molar ratio and 1 to 2 of the low-molecular-weight amine in terms of molar ratio, when the molar ratio of PCDA is set to 1.

In the case of a polymer obtained by adding polyallylamine to PCDA, as described in the temperature imaging device of the first embodiment of the present invention, the color change start temperature of the composite is reduced, and the color change temperature width thereof is increased as compared to those of the PCDA polymer free of any guest.

Meanwhile, when the low-molecular-weight amine is added to PCDA without addition of polyallylamine, the color change behavior (e.g., a width of the color change temperature and temperature responsiveness) of polydiacetylene varies depending on the low-molecular-weight amine. In addition, even in the case where polyallylamine is added to PCDA, when the low-molecular-weight amine is added as a third component, the color change behavior of the low-molecular-weight amine is exhibited. When the low-molecular-weight amine is a diamine, the color change may be reversible.

In the composite of poly-10,12-pentacosadiynoic acid (poly-PCDA), polyallylamine, and the low-molecular-weight amine, when a large amount of polyallylamine is inserted between the layers of poly-PCDA, the temperature imaging device becomes close to the temperature imaging device of the first embodiment of the present invention. When a large amount of the low-molecular-weight amine is inserted between the layers of poly-PCDA, phase separation between a portion formed of poly-PCDA and the low-molecular-weight amine and a portion formed of polyallylamine is liable to occur. According to the second embodiment of the present invention, a composite in which both of the coating property of poly-PCDA and polyallylamine, and the reversibility of the color change caused by the addition of the low-molecular-weight amine are achieved, and hence a temperature imaging device including the composite can be produced.

In the composite of poly-PCDA, polyallylamine, and the low-molecular-weight amine, as the amount of polyallylamine becomes larger, the color change start temperature is reduced as compared to the case where only poly-PCDA and the low-molecular-weight amine are used. In addition, when a diamine is incorporated as the low-molecular-weight amine, the reversibility of the color change may occur in a certain temperature range from the color change start temperature.

The color change start temperature, the color change end temperature, and the color change temperature width of the composite of poly-PCDA, polyallylamine, and the low-molecular-weight amine may vary depending on the kind of the low-molecular-weight amine. When, for example, p-xylenediamine is used as the low-molecular-weight amine, the color change start temperature is about 60°C, the color change end temperature is about 150°C, and the color change temperature width is about 90°C. Accordingly, the color change is so clear as to be observable with the naked eye within a practical temperature range.

Also in the temperature imaging device of the second embodiment, the composite of poly-10,12-pentacosadiynoic acid, polyallylamine, and the low-molecular-weight amine is used, and is hence suitable for an increase in area. When the composite is applied to a large-area substrate, in which the two-dimensional (length×width) dimensions of a surface to which the composite is applied are each 1 cm or more, by spray coating, uniform coating with the composite can be performed.

In some embodiments, in the temperature imaging device, the particle diameter of the composite may be observed with, for example, an electron microscope, and the composite may have a diameter of, for example, from 1 μm to 10 μm. The average particle diameter of the composite having a substantially spherical shape may also be measured, and composites each having a diameter of 1 μm or more observed with, for example, an electron microscope are randomly selected and the diameters of 20 or more composites are measured. An average particle diameter thereof may be from 1 μm to 10 μm.

The kind of the base material is as described for the temperature imaging device of the first embodiment. Base materials of various materials may each be used as the base material.

In some embodiments, the base material is preferably a woven fabric or a non-woven fabric from the viewpoint of the ease of adhesion of the composite to the base material. The particle size of the composite of the embodiment of the present invention is reduced as compared to that of a composite using a guest polymer except polyallylamine, and hence small particles easily adhere to a fiber material of the woven fabric or the non-woven fabric. Thus, uniform coating can be achieved. Accordingly, the composite is also suitable for large-area coating.

The shape and dimensions of the base material are also as described for the temperature imaging device of the first embodiment.

The temperature imaging device of the second embodiment of the present invention has a wide color change temperature range when the temperature is increased with the heating apparatus in the same manner as the temperature imaging device of the first embodiment. The color change of the temperature imaging device of the second embodiment of the present invention may be directly observed with the naked eye, or may be photographed to form an image and provided as numerical data.

The description for the temperature imaging device of the first embodiment may be applied to a preferred color change start temperature and a preferred color change end temperature of the temperature imaging device of the second embodiment of the present invention, and the temperatures may be varied by the low-molecular-weight amine to be used. In some embodiments, the color change occurs over the entire range of from at least 0°C to 100°C.

The temperature imaging device of the second embodiment of the present invention may also be used for various applications in each of which detection of a temperature change is required such as a method of examining a temperature change of a test site in the same manner as the temperature imaging device of the first embodiment.

In some embodiments, there is provided an energy device for a surgery including a coating caused to adhere to the energy device, the coating including a composite containing poly-10,12-pentacosadiynoic acid, polyallylamine, and an amine compound, the composite having at least part of the polyallylamine and/or the amine compound introduced between layers of poly-10,12-pentacosadiynoic acid. Examples of the energy device for a surgery are as described above.

As described above, in each of the temperature imaging device of the second embodiment of the present invention, and the heating apparatus including a coating caused to adhere to the heating apparatus, the coating including the composite of poly-10,12-pentacosadiynoic acid, polyallylamine, and the low-molecular-weight amine may be able to comprehensively solve the problem of thermal damage at the time of the use of the heating apparatus.

A method of producing a temperature imaging device of the second embodiment of the present invention includes:

applying, to a base material, a solution containing 10,12-pentacosadiynoic acid, polyallylamine having a unit represented by the following general formula (1), a low-molecular-weight amine, and a solvent by spray coating;
removing the solvent; and
polymerizing the 10,12-pentacosadiynoic acid to form a composite having at least part of the polyallylamine and/or the amine compound introduced between layers of poly-10,12-pentacosadiynoic acid:

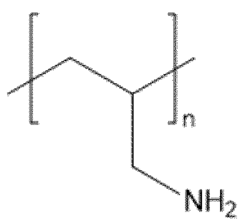

(1)

where "n" represents an integer.

The number-average molecular weight of the polyallylamine having the unit represented by the general formula (1) is preferably from 10,000 to 20,000.

The solvent of the solution to be used for the spray coating is not particularly limited, but is preferably an organic solvent. Examples thereof may include hexane, ethanol, and a mixed solvent thereof.

A ratio among PCDA, the polyallylamine having the unit represented by the general formula (1), and the low-molecular-weight amine in the solution is not particularly limited as long as a composite having the polyallylamine and/or the low-molecular-weight amine introduced between the layers of polydiacetylene can be formed, but the molar ratio of the amino group of the low-molecular-weight amine with respect to the carboxy group of PCDA is preferably from 0.6 to 1.0. In addition, the amount of the low-molecular-weight amine (e.g., p-Xyj) is preferably 0.4 mol or more, and the amount of the polyallylamine is preferably from 0.5 mol to 1.5 mol, more preferably from 1 mol to 1.3 mol, with respect to 1 mol of PCDA.

The dispersion of the solution, the application of the solution to the base material, the kind and shape of the base material, the removal of the solvent, the polymerization of 10,12-pentacosadiynoic acid, and the shape and dimensions of the composite to be obtained are as described for the method of producing a temperature imaging device of the first embodiment.

According to the method of producing a temperature imaging device of the second embodiment of the present invention by the spray coating, a desired substrate can be simply and uniformly coated, and hence a large-area device can be produced.

The present invention is more specifically described below by way of Examples, but the present invention is not limited thereto. In Examples, the term "PCDA" refers to 10,12-pentacosadiynoic acid before the polymerization, and the term "PDA" refers to polymerized PCDA unless otherwise stated.

## Examples

### Test 1 Production and Evaluation of Composite of PCDA and Polymer Guest

1. Production of PCDA and Polymer Guest

(1) Production of Mixed Solution of PCDA and Polymer Guest by Direct Synthesis Method

25 Milligrams of PCDA was dissolved in 2.5 ml of ethanol or 5 ml of hexane to produce a PCDA solution. The solution was filtered with a funnel and filter paper so that a polymer of PCDA was removed. 6.30 Milligrams of polyethyleneimine (PEI, Mw: about 25,000) or 37.4 μl of polyallylamine (PAAm, Mw: about 17,000)) was added to the filtered PCDA ethanol solution and 5.74 mg of polyvinylamine (PVAm, Mw: about 11,000) was added to the PCDA hexane solution so that the ratio "PCDA:guest" was 1:2.

The PCDA/alkyl-based polymer guest mixed solution was dispersed with an ultrasonic wave for 30 minutes, and then the solution was drop-cast onto a glass substrate. The solution was left to stand in a thermostatic bath at 4°C so that the solvent was volatilized and removed. Thus, a glass substrate device of the PCDA/alkyl-based polymer guest was obtained. The ratio represents a ratio at which the numbers of molecules of PCDA and the guest became equal to each other in terms of monomer unit of the guest.

(2) Production of PCDA/Pyridine-based Polymer Guest by Direct Synthesis Method

16 Milligrams of PCDA was dissolved in 1 ml of acetone to produce a PCDA solution. The solution was filtered with a funnel and filter paper so that a polymer of PCDA was removed. 414.0 Milligrams of poly(4-vinylpyridine) (PVP4, Mw: about 60,000)) or 215.1 mg of poly(2-vinylpyridine) (PVP2, Mw: about 37,500)) was added to the filtered PCDA solution so that the ratio "PCDA:guest" was 1:2.

3 Milliliters of ethanol and 2.7 ml of acetone were added to the PCDA/PVP4 or PCDA/PVP2 mixed solution, and 3.7 ml of acetone was added to the PCDA/PVP4 mixed solution. After that, the PCDA/pyridine-based polymer guest mixed solution was dispersed for 10 minutes with an ultrasonic wave until the solution became uniform, and was left to stand still for 1 day. The next day, 300 μl of the respective solutions dispersed for 10 minutes with an ultrasonic wave were drop-cast on two 2.5 cm square glass substrates on a cool plate at 40°C. As soon as the solvent was volatilized, 300 μl of the respective solutions were drop-cast on the same glass substrates. The procedure was continued until there was no solution. Thus, a glass substrate device of the PCDA/pyridine-based polymer guest was obtained.

(3) Production of PCDA/Alkyl-based Polymer Guest by Layer-by-layer Method (LbL Method)

Each of 4.73 mg of PEI, 4.29 mg of PVAm, and 28.0 μl of PAAm was dissolved in 5 ml of pure water to produce a guest solution. 18.7 Milligrams of PCDA was dissolved in 5 ml of acetone to produce a host solution. Each of a 0.5 cm square paper substrate and silicon substrate was immersed in the guest solution for 30 seconds and was then lifted. A surplus solution on the back surface and side surface of the substrate was removed with PROWIPE, and the substrate was subjected to natural drying at room temperature. Next, each of the substrates was washed by rinsing in pure water for from 2 seconds to 3 seconds and was dried in the same manner. Finally, each of the substrates was immersed in the host solution for 30 seconds and was then lifted, and was dried in the same manner. The procedure of immersion and washing with the three solutions and drying was defined as one cycle, and the cycle was repeated six times to provide a paper substrate device and a silicon substrate device of the PCDA/alkyl-based polymer guest.

The polymer guests of the items (1) to (3) were irradiated with UV light, and were subjected to solid-state photopolymerization. A hand lamp (AS ONE, Handy UV Lamp SLUV-6, 254 nm) was used as a light source.

2. Evaluation Method

(1) Observation of Color Change Behavior of Composite to Heat

With regard to a PDA/alkyl-based polymer guest and a PDA/alkyl-based low-molecular-weight guest after the polymerization, color change behavior to heat was observed. A glass substrate on which a sample was drop-cast or a paper substrate coated with the sample was placed on a temperature stage, and heating was performed from -50°C to a maximum of 220°C. The sample was kept for 2 minutes at every temperature, and was then photographed.

(2) X-ray Diffraction (XRD)

In order to observe the expansion of an interlayer distance and a change in periodic structure caused by formation of a composite with an amine, an X-ray diffraction pattern of a composite with the polymer guest before the polymerization was obtained by using X-ray diffraction (Bruker, D8 Advance). The voltage and current value of an X-ray tube bulb were set to 40 kV and 40 mA, respectively, and measurement was performed by a continuous scanning method with a CuKα ray.

(3) Scanning Electron Microscope (SEM)

The glass substrate on which the PCDA/polymer guest was drop-cast and polymerized was fixed to an aluminum stage with a conductive tape. Osmium coating was performed to secure conductivity. After that, a detailed structure of the sample was observed with a scanning electron microscope (S-2700; Hitachi, Sirion; FEI, JSM-7600F; Jeol).

(4) Optical Microscope

The structure of the PDA/polymer guest on the silicon substrate was observed with a fluorescent microscope (OLIMPUS, BX51-FL).

(5) Ultraviolet-visible Spectroscopy (UV-Vis)

In the observation of the temperature color change behavior of each of PDA and PDA/PAAm, measurement was performed by using, as a sample, the glass substrate on which each of PCDA and PCDA/PAAm was drop-cast and polymerized. A halogen lamp included with an optical microscope (Keyence, VHX-1000) was used as a light source, and transmitted light from the sample fixed onto a temperature control stage was detected with a spectrophotometer (Hamamatsu photonics, PMA-12) to provide a UV-Vis spectrum.

(6) Fourier Transform Infrared Spectroscopy (FT-IR)

In order to observe an interaction between PCDA and each of the guest polymers, and in order to observe a change in interaction before and after the color change, an infrared absorption spectrum was obtained with a Fourier transform infrared spectrophotometer (Jasco, FT/IR-4200, Shimadzu, AIM-8800) by using a KBr method and an ATR method.

(7) Differential Scanning Calorimeter (DSC)

In order to analyze the thermal behavior of the PDA/alkyl-based polymer guest, measurement was performed with a differential scanning calorimeter (Shimadzu Science East Corporation, DSC-60 Plus).

(8) Raman Spectrometer

In order to compare polymerization rates of the PCDA/polymer guests, measurement was performed with a Raman spectrometer (Renishaw, InVia Raman Microscope). Measurement was performed by bonding a carbon tape to an upper lid of a styrol square box, and fixing powder of the sample onto the tape.

(9) Image Analysis (Chromaticity Diagram)

Color changes of the PDA/polymer guests were quantitatively analyzed by image analysis. RGB value analysis of the photograph of the composite that was taken was performed with image analysis software (Image J). The result was converted to a CIE 1931 color system based on the determined RGB values. The equation used for the conversion is shown below.

$$\begin{bmatrix} X \\ Y \\ Z \end{bmatrix} = \begin{bmatrix} 2.7689 & 1.7517 & 1.1302 \\ 1 & 4.5907 & 0.0601 \\ 0 & 0.0565 & 5.5943 \end{bmatrix} \begin{bmatrix} R \\ G \\ B \end{bmatrix}$$

3. Result

(1) Production of PCDA/Alkyl-based and Pyridine-based Polymer Guests and Evaluation of Composite

PCDA/polymer guests were produced by a direct synthesis method using PEI, PVAm, and PAAm as alkyl-based polymers, and using PVP4 and PVP2 as pyridine-based polymers. The produced composites before polymerization were evaluated based on the analysis of the XRD pattern and the IR spectrum.

XRD patterns of a PCDA crystal and PCDA/PEI, PCDA/PVAm, and PCDA/PAAm each serving as the PCDA/alkyl-based polymer guest are shown in Fig. 3(A). Peaks were able to be observed at 2θ/θ=1.85°, 3.65°, 7.37°, and 13.0° in the PCDA crystal, and were able to be attributed to d0=4.68 nm. After the formation of the composite with the guest polymer, peaks were able to be observed at 2θ/θ=1.71°, 2.48°, and 5.04° in PCDA/PEI, at 2θ/θ=1.67°, 5.10°, 8.27° in PCDA/PVAm, and at 2θ/θ=1.28° in PCDA/PAAm, and the peaks were able to be attributed to d0=5.51 nm, 5.63 nm, and 7.05 nm, respectively. Accordingly, it was found that the guest polymer was introduced between PCDA layers because a shift in a peak indicating an interlayer distance was observed when the composite with the guest polymer was formed.

IR spectra of the PCDA crystal and the PCDA/alkyl-based polymer guests are shown in Fig. 3(B). In the precursor PCDA crystal, an absorption peak (A) derived from the C=O stretching vibration of a carboxy group (-COOH) that formed a dimer through hydrogen bonding was observed around 1,700 $cm^{-1}$. Meanwhile, in each of the PCDA/alkyl-based polymer guests, the absorption peak around 1,700 $cm^{-1}$ disappeared, and an absorption peak (B) derived from the C=O stretching vibration of a carboxylate anion ($-COO^-$) was observed at from 1,550 $cm^{-1}$ to 1,650 $cm^{-1}$. It is found from the foregoing that the carboxy group (-COOH) at a terminal formed a dimer in the precursor PCDA crystal, but became a carboxylate anion ($-COO^-$) through the formation of a composite with an amine in each of the PCDA/alkyl-based polymer guests.

XRD patterns of the PCDA crystal and the pyridine-based polymer guests are shown in Fig. 4(A). Peaks were able to be observed at 2θ/θ=1.85°, 3.65°, 7.37°, and 13.0° in the PCDA crystal, and were able to be attributed to d0=4.68 nm. After the formation of the composite with the guest polymer, peaks were able to be observed at 2θ/θ=1.77° and 5.74° in PCDA/PVP4 and at 2θ/θ=1.64° in PCDA/PVP2, and the peaks were able to be attributed to d0=4.88 nm and 5.44 nm, respectively. Accordingly, it was found that the guest polymer was introduced between PCDA layers because a shift in a peak indicating an interlayer distance was observed when the composite with the guest polymer was formed.

IR spectra of the PCDA crystal and the PCDA/pyridine-based polymer guests are shown in Fig. 4(B). In the precursor PCDA crystal, an absorption peak (A) derived from the C=O stretching vibration of a carboxy group (-COOH) that formed a dimer through hydrogen bonding was observed around 1,700 $cm^{-1}$. Meanwhile, in each of the PCDA/pyridine-based polymer guests, the absorption peak around 1,700 $cm^{-1}$ was shifted to a low wavenumber side. It was found from the foregoing that precursor PCDA molecules that formed a dimer through hydrogen bonding of the carboxylic acid were monomerized in the PCDA/pyridine-based polymer guest. In addition, with regard to an absorption peak (B) around 1,600 $cm^{-1}$ derived from the stretching vibration of a pyridine ring, an absorption peak of the PCDA/pyridine-based polymer guest was shifted to a high wavenumber side as compared to an absorption peak of the pyridine-based polymer guest. This

indicates that the hydrogen bonding became strong. It was found from the above-mentioned results that the monomerized precursor PCDA and the pyridine-based polymer guest interacted with each other through the hydrogen bonding.

When XRD patterns and IR spectra were measured by setting the composition ratio "PCDA:pyridine-based polymer guest" to 1:3, 1:4, 1:5, and 1:10 in addition to 1:2 to produce samples (data not shown), PCDA and the pyridine-based polymer guest formed composites at all the composition ratios. Accordingly, it was inferred that the amount of an interlayer guest did not change when the ratio "PCDA:pyridine-based polymer guest" was 1:2 or more. The evaluation was hereinafter performed only on samples in each of which the ratio "PCDA:pyridine-based polymer guest" was 1:2.

(2) Changes of Composites of PCDA/Alkyl-based and Pyridine-based Polymer Guests after UV Irradiation

Photographs of PDA/polymer guests obtained by irradiating PCDA/each of five kinds of polymer guests with UV light to perform topochemical polymerization are shown in Figs. 5. In each of Fig. 5(E) and Fig. 5(F), the color change was insufficient. It is conceived from the foregoing that polymerization did not sufficiently progress in PDA/PVP4 and PDA/PVP2.

Changes in peak intensity ratios of the respective composites with respect to UV irradiation time, that is, polymerization rates are shown in Fig. 6 (the peak intensity is based on a peak of a Raman spectrum measured with a Raman spectrometer).

Peak intensity ratio=intensity of peak derived from carbon-carbon triple bond of enyne skeleton/intensity of peak derived from carbon-carbon triple bond of monomer

As shown in Fig. 6, PCDA had the fastest polymerization rate, the alkyl-based polymer guest composites each had the second fastest polymerization rate, and PCDA/PVP4 and PCDA/PVP2 serving as the pyridine-based polymer guest composites each had the slowest polymerization rate. The PCDA molecules are required to be aligned at an angle and an intermolecular distance that allow polymerization for the polymerization because PCDA has a property that undergoes topochemical polymerization. However, the pyridine-based polymer guest is extremely bulky, and hence largely increases the flexibility of the composite. Accordingly, an alignment property required for polymerization cannot be kept, and the polymerization rate becomes slow. It is conceived from the foregoing that polymerization did not sufficiently progress.

Accordingly, the evaluation was hereinafter performed only on temperature-responsive color changes of PDA/PEI, PDA/PVAm, and PDA/PAAm serving as composites of the alkyl-based guests in which polymerization sufficiently progressed.

(3) Temperature Responsiveness of PDA/Alkyl-based Polymer Guest

Each of PDA, PDA/PEI, PDA/PVAm, and PDA/PAAm polymers was gradually heated from low temperature, and color changes at the respective temperatures were photographed. Color change photographs are shown in Fig. 7.

It is found from the color change temperature ranges indicated by the arrows in Fig. 7 that the color change start temperature of the PDA/alkyl-based polymer guest is reduced and the color change temperature width thereof is increased as compared to those of PDA. In order to quantitatively evaluate the color change, image analysis was performed on Fig. 7. First, the color changes of the respective samples at the respective temperatures were plotted on the chromaticity diagram (Fig. 8(A)). Next, distances between the measurement starting points of the respective samples and the respective plots were calculated. The distances between the measurement starting points and the respective plots with respect to a temperature increase are shown in Fig. 8(B).

The color change temperature ranges determined based on Fig. 8(B) are shown in Table 1.

It was found from Table 1 that the color change start temperature of the PDA/alkyl-based polymer guest was reduced and the color change temperature width thereof was increased as compared to those of PDA. In addition, as the bulkiness of the guest polymer increased, the color change temperature of the PDA/alkyl-based polymer guest was more reduced and the color change temperature width thereof is more increased. With regard to PDA/PAAm that showed the maximum reduction in temperature and the maximum increase in width, further temperature responsiveness evaluation was performed by UV-vis measurement. The results of the UV-vis measurement are shown in Fig. 9(A) and Fig. 9(B). PDA and PDA/PAAm were each heated from -50°C to 220°C and measured at the respective temperatures. The absorption peak of PDA was sharply shifted to a low wavelength side around 60°C. Meanwhile, the absorption peak of PDA/PAAm was gradually shifted from low temperature to a low wavelength side in response to the temperature change. Accordingly, it was also found from the results of the UV-vis measurement that PDA/PAAm exhibited a specific color change caused by the introduction of the guest polymer.

Table 1. Color change temperature ranges of PDA and PDA/Alkyl-based polymer guest

| T/°C | Color change start temperature | Color change end temperature | Color change temperature width |
|---|---|---|---|
| PDA | 70 | 110 | 40 |
| PDA/PEI | 30 | 80 | 50 |
| PDA/PVAm | -20 | 120 | 140 |
| PDA/PAAm | -50 | 220 | 270 |

The color change is caused by torsion of a PDA main chain. Accordingly, from a graph that shows a torsion angle of the PDA main chain with respect to the maximum absorption wavelength, the ease of disturbance of the PDA layer, that is, flexibility of the composite can be estimated from the color change. Torsion angles of conjugated chains of PDA and PDA/PAAm at the respective temperatures were determined from the UV-vis spectra of each of Fig. 9(A) and Fig. 9(B) and the graphs (Fig. 10(A) and Fig. 10(B)).

As shown in Fig. 10(A), the conjugated chain of PDA sharply formed torsion at from 60°C to 70°C. In addition, as shown in Fig. 10(B), the conjugated chain of PDA/PAAm gradually formed torsion along with a temperature increase. There was observed torsion angles of the conjugated chains corresponding to the color changes of PDA and PDA/PAAm. The range of the torsion angle was from 3° to 35° for PDA, and from 15° to 47° for PDA/PAAm. When the maximum values of the torsion angles are compared to each other, it is conceived that PDA/PAAm had higher flexibility of the layer structure than that of PDA.

Measurement results of the PDA/alkyl-based polymer guests with a DSC are shown in Fig. 11. The arrow in Fig. 11 indicates a start temperature of an endothermic peak. As the bulkiness of the guest polymer became larger, the start temperature of the endothermic peak was reduced. The endothermic peak of the DSC appears through the molecular motion of the composite, that is, the disturbance of the layer structure. The color change of the composite is caused by the torsion of the PDA main chain by the disturbance of the layer structure. It is conceived from the foregoing that: the reduction in color change temperature was achieved because the flexibility of the composite was increased as the bulkiness of the guest polymer became larger and hence the disturbance of the layer structure was easily caused by a small heat stimulus, that is, from a low temperature; and the color change temperature width was increased because the layer structure was gradually disturbed.

(4) Comparison between Methods of producing PCDA/Alkyl-based Polymer Guest Composite

The sample of the PCDA/alkyl-based polymer guest composite was produced by the direct synthesis method in the previous sections. In this section, however, a sample was produced by a layer-by-layer method (LbL method) in expectation of lamination by a stronger electrostatic interaction. The production was performed by the LbL method. Polymerization of the PCDA/alkyl-based polymer guest composite was performed by irradiation with UV light (254 nm).

The produced composite before the polymerization was evaluated by the analysis of the XRD pattern. As a result, peaks were able to be observed at 2θ/θ=1.85°, 3.65°, 7.37°, and 13.0° in the PCDA crystal, and were able to be attributed to d0=4.68 nm. After the introduction of the guest polymer, peaks were able to be observed at 2θ/θ=1.73°, 2.12°, 5.10°, and 5.25° in PCDA/PEI, at 2θ/θ=1.71°, 5.06°, and 5.53° in PCDA/PVAm, and at 2θ/θ=1.48° and 5.62° in PCDA/PAAm, and the peaks were able to be attributed to d0=5.08 nm, 5.26 nm, and 6.40 nm, respectively (graph omitted). Accordingly, it was found that the guest polymer was introduced between PCDA layers because a shift in a peak indicating an interlayer distance was observed when the composite with the guest polymer was formed.

In addition, according to the analysis of the IR spectrum, an absorption peak (A) around 1,700 $cm^{-1}$ derived from the C=O stretching vibration of a carboxy group (-COOH) that formed a dimer was not observed in the PCDA/alkyl-based polymer guest composite, and an absorption peak (B) derived from the C=O stretching vibration of a carboxylate anion ($-COO^-$) was observed at from 1,550 $cm^{-1}$ to 1,650 $cm^{-1}$ (graph omitted). It is found from the foregoing that a carboxy group (-COOH) at a terminal of the host layer PCDA became a carboxylate anion ($-COO^-$) by formation of a composite with an amine of the guest polymer.

Thus, it was recognized that PCDA and the alkyl-based polymer guest formed a composite in the sample produced by the LbL method as well as the sample produced by the direct synthesis method.

Also in color photographs obtained when the respective polymer samples were gradually heated from a low temperature and the color changes at the respective temperatures were photographed, no significant difference was found as compared to the color change behavior of the sample produced by the direct synthesis method as shown in Fig. 7 (data not shown). The reduction in color change temperature and the increase in width thereof were observed in the same manner as in the direct synthesis method even when the sample was produced by the LbL method.

The structures of the respective composites produced and polymerized by the LbL method were observed with an optical microscope (Fig. 12(A) to Fig. 12(C)) and a SEM (Fig. 13(A) to Fig. 13(C)). In PDA/PEI, the amount of the sample

formed on the substrate was small, and in each of PDA/PVAm and PDA/PAAm, the sample formed on the substrate was nonuniform. A possible factor of such nonuniformity is, for example, as follows: that aggregation of host molecules and of guest molecules randomly occurred on the substrate in a process of alternately laminating the host solution and the guest solution, and hence the thickness per layer became nonuniform.

(5) Observation of PDA/PAAm Coating by Various Coating Methods

A silk cloth was coated with the PDA/PAAm composite by various coating methods, and the state of the coating was observed with a typical camera and an optical microscope.

Fig. 14(A) and Fig. 14(D) are a photograph taken with the camera of the silk cloth coated with the PDA/PAAm composite by a dip coating method and an optical micrograph thereof. Solution conditions were set to be the same as those in 1.(1) of Test 1. The silk cloth was immersed in the PDA/PAAm solution, and the silk cloth was lifted and dried.

Fig. 14(B) and Fig. 14(E) are a photograph taken with the camera of the silk cloth coated with the PDA/PAAm composite by a volatilization method and an optical micrograph thereof. Solution conditions were set to be the same as those in 1. (1) of Test 1. The solution was evaporated under a state in which the silk cloth was immersed in the PDA/PAAm solution.

Fig. 14(C) and Fig. 14(F) are a photograph taken with the camera of the silk cloth coated with the PDA/PAAm composite by a spray coating method and an optical micrograph thereof. A solution of the PCDA/PAAm composite was applied to the silk cloth by spray coating from a position distant therefrom by 20 cm. The silk cloth was left to stand in a thermostatic bath at 4°C so that the solvent was volatilized and removed. The PCDA/PAAm composite of the obtained silk cloth was subjected to solid-state photopolymerization by irradiation with UV light. A UV-LED lamp (CCS Inc., HLDL-120X30UV265-PSC, 265 nm) or a hand lamp (AS ONE, Handy UV Lamp SLUV-6, 254 nm) was used as a light source.

In the case of each of the dip coating method and the volatilization method, the color of the silk cloth was light, and the PDA/PAAm composite did not uniformly adhere to the silk cloth. In the case of the spray coating method (Fig. 14(C) and Fig. 14(F)), the PDA/PAAm composite uniformly adhered to the silk cloth.

<u>Test 2 Increase in Area of PDA/PAAm Composite, Measurement of Specific Heat thereof, and Application thereof to Medical Surgery</u>

There have been reports of research examples of an increase in area of a polydiacetylene device by film device formation and inkjet printing. Most of the research examples of the increase in area are examples of inkjet printing on a self-standing film or paper, research in a field of uniform coating on various substrates on a centimeter scale is underdeveloped. When such coating is enabled, the application can be expanded in applications such as temperature imaging in various situations.

In this Example, large-area substrates of the PDA/alkyl-based polymer guests were produced, followed by comparison in coating performance at a centimeter scale. An attempt was made to estimate a value equivalent to a specific heat with a bondable seal substrate and to perform real-time temperature imaging with a silk cloth substrate by utilizing the color change characteristic and the coating performance of

PDA/PAAm.

1. Experiment Method

(1) Application of PCDA/Polymer Guest to Polymethyl Methacrylate (PMMA) Substrate by Spray Coating

80 Milligrams of PCDA was dissolved in 8 ml of ethanol or 16 ml of hexane to produce a PCDA solution. The solution was filtered with a funnel and filter paper so that a polymer of PCDA was removed. 0.5 Milliliter of ethanol and 20.2 mg of PEI or 119.7 μl of PAAm were added to the filtered PCDA ethanol solution, and 1 ml of hexane and 5.74 mg of PVAm were added to the filtered PCDA hexane solution. The PCDA/alkyl-based polymer guest mixed solution was dispersed with an ultrasonic wave for 30 minutes, and then the solution was applied to an 18 cm×18 cm PMMA substrate in an upright state by spray coating from a position distant from the substrate by 20 cm in order from the bottom thereof. The substrate was left to stand in a thermostatic bath at 4°C so that the solvent was volatilized and removed. Thus, a PMMA substrate device of the PCDA/alkyl-based polymer guest was obtained. The obtained PCDA/alkyl-based polymer guest composite was subjected to solid-state photopolymerization by irradiation with UV light. A UV-LED lamp (CCS Inc., HLDL-120X30UV265-PSC, 265 nm) or a hand lamp (AS ONE, Handy UV Lamp SLUV-6, 254 nm) was used as a light source.

(2) Drop Casting of PDA/PAAm on Bondable Seal

50 Milligrams of PCDA was dissolved in 5 ml of ethanol to produce a PCDA solution. The solution was filtered with a funnel and filter paper so that a polymer of PCDA was removed. 74.8 Microliters of PAAm was added to the filtered PCDA ethanol solution, and was dispersed with an ultrasonic wave for 30 minutes. The solution was drop-cast on a 3.5 cm×7.5 cm bondable seal (a seal including a transparent sheet made of polyvinyl chloride having an adhesive on one surface of the sheet, the seal being used by peeling release paper on the adhesive and being bonded to an object) that was fixed to a smooth plate with a masking tape. The seal was left to stand in a thermostatic bath at 4°C so that the solvent was volatilized and removed. Thus, a bondable seal device of PCDA/PAAm was obtained. The obtained PCDA/PAAm composite was subjected to solid-state photopolymerization by irradiation with UV light. A UV-LED lamp (CCS Inc., HLDL-120X30UV265-PSC, 265 nm) or a hand lamp (AS ONE, Handy UV Lamp SLUV-6, 254 nm) was used as a light source.

(3) Application of PDA/PAAm to Silk Cloth by Spray Coating

40 Milligrams of PCDA was dissolved in 12 ml of ethanol to produce a PCDA solution. The solution was filtered with a funnel and filter paper so that a polymer of PCDA was removed. 59.8 Microliters of PAAm was added to the filtered PCDA ethanol solution, and was dispersed with an ultrasonic wave for 30 minutes. Four 3 cm×3 cm silk cloths were each fixed to a smooth plate with a masking tape, and half an amount of the solution was applied to the plate by spray coating from above the plate by 20 cm. The plate was left to stand in a thermostatic bath at 4°C so that the solvent was volatilized. The next day, the remaining solution was applied to the plate by spray coating. The plate was left to stand in a thermostatic bath at 4°C so that the solvent was volatilized. Thus, a silk cloth device of PCDA/PAAm was obtained. The obtained PCDA/PAAm composite was subjected to solid-state photopolymerization by irradiation with UV light. A UV-LED lamp (CCS Inc., HLDL-120X30UV265-PSC, 265 nm) or a hand lamp (AS ONE, Handy UV Lamp SLUV-6, 254 nm) was used.

(4) Observation of Color Change Behavior of Silk Cloth coated with PDA/PAAm to Heat

The color change behavior of the silk cloth coated with PDA/PAAm to heat was observed. The glass substrate on which the sample was drop-cast or the paper substrate coated with the sample was placed on a temperature stage, and heating was performed at from 30°C to 120°C. The sample was kept for 2 minutes at every temperature, and was then photographed.

2. Evaluation Method

(1) Scanning Electron Microscope (SEM)

A glass substrate on which a PDA/polymer guest was drop-cast was fixed to an aluminum stage with a conductive tape. Osmium coating was performed to secure conductivity. After that, a detailed structure of the sample was observed with a scanning electron microscope (S-2700; Hitachi, Sirion; FEI, JSM-7600F; Jeol).

(2) Image Analysis (Vertical Axis "x")

A color change of PDA/APDES caused by a frictional force was quantitatively analyzed by image analysis. R, G, and B values were calculated from a photograph with software (ImageJ). The results were converted to an XYZ color system through use of Rec.709 standard. The equation used for the conversion is shown below.

$$\begin{bmatrix} X \\ Y \\ Z \end{bmatrix} = \begin{bmatrix} 0.4124 & 0.3576 & 0.1805 \\ 0.2126 & 0.7152 & 0.0722 \\ 0.0193 & 0.1192 & 0.9505 \end{bmatrix} \begin{bmatrix} R \\ G \\ B \end{bmatrix}$$

$$x = \frac{X}{X + Y + Z}$$

3. Results

Photographs of an ethanol solution of PDA and ethanol precursor solutions of PCDA/polymer guests each applied to a polymethyl methacrylate (PMMA) substrate by spray coating and polymerized by UV irradiation are shown in

Fig. 15(A) to Fig. 15(D). As the bulkiness of the guest polymer increased, macro uniformity of the coating increased.

SEM images of the respective substrates of Fig. 15(A) to Fig. 15(D) are shown in Fig. 16(A) to Fig. 16(D). Also in a micro scale, uniformity of the coating increased as the bulkiness of the guest polymer increased. The forms of the respective composites were observed from the SEM images. The form of PDA was a plate-like crystal having a thickness of 0.20±0.025 μm and a size of 17.52+6.25 μm×12.25±4.26 μm, the form of PDA/PEI was an amorphous shape having a thickness 0.34 μm and a size of 29.81+8.99 μm×9.00±2.81 μm, the form of PDA/PVAm was a belt shape having a width of 0.22+0.14 μm and a length 5.54+2.43 μm, and the form of PDA/PAAm was a spherical shape having a particle diameter of 2.07±1.04 μm. As the bulkiness of the guest polymer increased, the size of the composite reduced. A possible factor therefor is as follows: as the guest polymer became bulkier, a crystal of guest molecules was hardly formed and a large amount of the guest was present around the PDA crystal, and hence the crystal growth of PDA was inhibited. In PDA/PAAm using the most bulky guest polymer, most uniform coating with a large area was enabled.

4. Estimation of Value equivalent to Specific Heat utilizing PDA/PAAm

An attempt was made to estimate a value equivalent to a specific heat through use of a bondable seal coated with PDA/PAAm. The bondable seal coated with PCDA/PAAm was bonded to a side surface of a container made of stainless steel, and 100 g of a liquid serving as a measurement object was loaded into the container. In order to prevent a mass change through evaporation of the liquid, the container was lidded with a plastic wrap, and was sealed with a masking tape. The container was left to stand still on a hot stage at 70°C to be heated, and the appearance thereof was videographed. During the heating, the container was enclosed and an upper portion of the hot stage was covered with a styrofoam box so as not to be influenced by, for example, a temperature change of a surrounding environment. A hole was made in the styrofoam box for videographing, and the hole was sealed with a transparent polymethyl methacrylate plate. The inside of the box becomes dark, and hence the PDA/PAAm-coated seal on the side surface of the container was illuminated with an LED desk light from the outside of the box so that a color change of PDA/PAAm was accurately videographed.

A video was taken for each of the cases where 100 g of silicone oil, ethanol, and water were loaded into the container as solutions serving as measurement objects by the above-mentioned method. For the image analysis of the taken video, the video was played on a personal computer, and was paused to be screenshot at the respective heating times, and an image of only a PDA/PAAm part was cut out with a trimming function of PowerPoint. As a result of the image analysis, rates of progress of the color change were compared to each other in a graph at a heating time before a steady state of a color change of the seal, and it was found that a color change corresponding to a literature value of a specific heat was shown.

The definition formula of a specific heat "c" is as described below.

$$\bar{c} = \frac{\Delta Q}{m \Delta T}$$

In the formula, it is conceived that a difference ΔQ in thermal energy of an object corresponds to a total time period $t_{total}$ of heating of the hot stage at a constant temperature of 70°C from the start of heating of the hot stage until the color change becomes plateau. In addition, it is conceived that a temperature change ΔT corresponds to Δx0→max from the following equation.

$$\Delta T = T_{\text{end of temperature increase}} - T_{\text{start of heating}} = x_{\text{color change becomes plateau}} - x_{\text{heating time of 0 s}} = \Delta x_{0 \rightarrow max}$$

Thus, a specific heat equivalent value c' is represented by the following equation.

$$c' = \frac{t_{total}}{m \Delta x_{0 \rightarrow max}}$$

A relationship between a specific heat equivalent value of each of the solutions serving as measurement objects measured from an inclination of an approximate straight line of the graph at the heating time before the steady state of the color change, and a value of a liquid having the same specific heat of the literature is shown in Fig. 17. The calibration curve that allowed estimation of the specific heat from the specific heat equivalent value was determined as the following equation. "c" represents a specific heat, and c' represents a specific heat equivalent value.

$$c = 8.93c' \times 10^{-3} - 1.10$$

A specific heat of an unknown solution can be estimated by using the equation as a calibration curve. For example, when a container filled with 100 g of a 50 wt% ethanol aqueous solution was heated, a color change image of the PDA/PAAm seal on the side surface of the container was taken, and a relationship between the calculated specific heat equivalent value and the specific heat of the literature value was determined. As a result, the relationship corresponded to the calibration curve (the point (d) of Fig. 17).

5. Temperature Imaging of Medical Surgery utilizing PDA/PAAm

An attempt was made to perform real-time temperature imaging for preventing a burn caused by an electric scalpel in a medical surgery. A temperature imaging sheet obtained by applying a PCDA/PAAm mixed solution to a silk cloth by spray coating was used.

A temperature at which the burn occurs, that is, a temperature at which protein denaturation occurs is around 60°C, and is in a color change temperature region of a sheet containing a PDA/PAAm polymer composite. Accordingly, a site in a temperature range of 60°C or more can be detected while attention is paid to a color change of the temperature imaging sheet.

On the assumption that the temperature imaging sheet is attached to an organ during a surgery, as shown in Fig. 18, the temperature imaging sheet was attached to a piece of pork, and the piece of pork was subjected to heat treatment with the electric scalpel. Accordingly, a color change around the treatment site was analyzed with an image.

Fig. 19(A) is a photograph of cutting of a piece of meat utilizing an ultrasonic wave on the outer side (a curved side of a blade) of an electric scalpel A (an ultrasonic coagulation incision apparatus to be cut by ultrasonic vibration). Fig. 19(B) is a photograph of cutting of the piece of meat utilizing electricity on the outer side of an electric scalpel B (vessel sealing system including cutting with Joule heat). Fig. 19(C) is a graph showing relationships between a distance "z" from a cut site of the piece of meat (horizontal axis), and a red intensity ("x", left axis) of the sheet and an estimated temperature (right axis) when the electric scalpels A and B are used. Fig. 19(D) is a calibration curve showing a relationship between the red intensity ("x", left axis) of the sheet and an actually measured temperature (right axis) of the sheet.

The distance "z" from the cut site of the piece of meat is a distance from an edge of the cut piece of meat to a measurement site on the piece of meat in a direction distant from the cut site as shown in each of Fig. 19(A) and Fig. 19(B). A red intensity "x" of the sheet was calculated by image analysis with an image captured from a photograph of the sheet. R, G, and B values were calculated with software (ImageJ) from the image of the sheet, and were converted to an XYZ color system through use of Rec.709 standard. The equation used for the conversion is shown below.

$$\begin{bmatrix} X \\ Y \\ Z \end{bmatrix} = \begin{bmatrix} 0.4124 & 0.3576 & 0.1805 \\ 0.2126 & 0.7152 & 0.0722 \\ 0.0193 & 0.1192 & 0.9505 \end{bmatrix} \begin{bmatrix} R \\ G \\ B \end{bmatrix}$$

$$x = \frac{X}{X + Y + Z}$$

An estimated temperature T of the sheet was calculated from the red intensity "x" of the sheet and the calibration curve of Fig. 19(D). An average value of the distances "z" from the cut site of the piece of meat indicated to be 60°C or more by the analysis of the color of the temperature imaging sheet when the sheet was cut three times in the case where the electric scalpel A was used was 1.32±0.405 mm. The average value of the distance "z" when the sheet was cut three times in the case where the electric scalpel B was used was 2.34±0.698 mm. It is understood that, when the electric scalpel B was utilized, a site at 60°C or more spread over a wide range from the cut site, and hence an influence range of heat is wider than that of the electric scalpel A. Meanwhile, the maximum attainable temperature of the temperature imaging sheet was higher when an ultrasonic wave was utilized with the electric scalpel A, than when electricity was utilized with the electric scalpel B. The same result was obtained even when the sheet was cut with the inner side of the electric scalpel A (data omitted).

Test 3 Production and Evaluation of Composite of PCDA, PAAm, and Low-molecular-weight Amine

(1) Production of PCDA/Guest Composite

30 Milligrams of PCDA was dissolved in 20 mL of a solvent to produce a PCDA solution. The solution was filtered with a funnel and filter paper so that a polymer of PCDA was removed. Each of four kinds of low-molecular-weight amines serving as guests (all of p-xylenediamine, 1,4-bis(aminomethyl)cyclohexane (c-$C_6$-$(CH_2NH_2)_2$ (mixture of cis- and trans-isomers)), 1,12-diaminododecane ($C_{12}$-$(NH_2)_2$), and stearylamine ($C_{18}$-$(NH_2)$)) was added to the filtered solution so that a ratio between the amounts of the cation moiety of each of the guests and the carboxy group of PCDA became 1:1. After ultrasonic stirring was performed for 30 minutes, the solvent was removed through solvent volatilization from the produced precursor solution. Thus, a PCDA/guest composite was obtained. The obtained PCDA/guest composite was subjected to solid-state photopolymerization by irradiation with UV light. A UV-LED lamp (CCS Inc., HLDL-120X30UV265-PSC, 265 nm) or a hand lamp (AS ONE, Handy UV Lamp SLUV-6, 254 nm) was used as a light source.

*p*-xylylenediamine
(*p*-Xy)

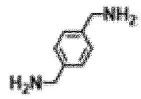

1,4-Bis(aminomethyl)cyclohexane
(c-$C_6$-$(NH_2)_2$)

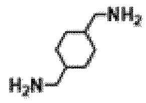

1,12-Diaminododecane
($C_{12}$-$(NH_2)_2$)

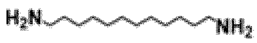

stearylamine
($C_{18}$-$NH_2$)

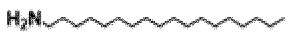

Four kinds of low-molecular-weight amines

(2) Production of PAAm/PDA-Guest Phase Separation Structure

(2-1) Investigation on Change in Introduction Amount of PAAm and Reversibility of Color Change

30 Milligrams of PCDA was dissolved in 20 mL of a solvent to produce a PCDA solution. The solution was filtered with a funnel and filter paper so that a polymer of PCDA was removed. Each of guest molecules that were low-molecular-weight amines was added to the filtered solution so that a ratio between the amounts of the cation moiety of each of the above-mentioned four kinds of low-molecular-weight amines and the carboxy group moiety of PCDA became 1:1. After ultrasonic stirring was performed for 30 minutes, any amount of PAAm was added, and the resultant was further subjected to ultrasonic stirring for 60 minutes. The solvent was removed through solvent volatilization from the produced precursor solution, and the resultant was subjected to UV irradiation. Thus, a PAAm/PDA/guest phase separation structure was obtained.

Various evaluation methods below were performed in the same manner as in Test 1 unless otherwise specified.

When the structure of polyallylamine having a unit represented by the general formula (1) was set to one unit, an introduction amount of PAAm was defined as a molar ratio with respect to PCDA in the sample, and an introduction amount of p-xylenediamine (hereinafter referred to as "p-Xy") was defined as a molar ratio of PCDA.

p-Xy was set to 0.5, and a color change at the time of temperature increase from 25°C to 118°C when the introduction amount of PAAm was changed in a range of from 0 to 2 (0, 0.5, 1, 1.33, 1.66, and 2), and reversibility at the time of cooling to 20°C were evaluated. There is no color reversibility in PDA-PAAm. As shown in Fig. 20(A), the color change at the time of the temperature increase did not largely depend on the introduction amount of PAAm. In addition, as is understood through comparison between Fig. 20(A) and Fig. 20(B), when the amount of PAAm was large, there was a tendency that the reversibility was poor. It was found that the case in which the introduction amount of PAAm was 1.33 or less was more preferred from the viewpoint of reversibility.

(2-2) Investigations on Change in Amount of p-Xy and Reversibility of Color Change

Next, in the case where the ratio between PDA and PAAm was fixed to 1:1 in terms of molar ratio and the amount of p-Xy was changed within the range of from 0.2 to 0.5 (molar ratio with respect to PCDA), a color change at the time of

temperature increase from 25°C to 118°C and reversibility at the time of cooling to 20°C were evaluated. In Fig. 21, the solid lines indicate color changes of composites increase containing p-Xy in three kinds of amounts at the time of temperature increase, and the dotted lines indicate color changes of the composites containing p-Xy in three kinds of amounts at the time of cooling. A larger arrow on the right side of the graph indicates that the reversibility of the color change is larger. In this experiment, the largest reversibility was shown when p-Xy was 0.5.

(2-3) Investigation on Introduction Distribution of p-Xy and PAAm

Subsequently, an experiment of changing the introduction distribution of p-Xy and PAAm was performed. When the number of carboxy groups of PCDA was set to 1, the number of amino groups that was present was fixed to 1, five kinds of PAAm/PDA-p-Xy were produced at mixed ratios as shown in Table 2. The IR measurement results of the respective samples are shown in Fig. 22. According to Fig. 22, PAAm/PDA-p-Xy and PDA-p-Xy showed similar peaks without depending on the amounts of p-Xy and PAAm. In addition, images of composites of samples II, III, and IV were taken under an optical microscope, and it was found from Fig. 23(A) to Fig. 23(C) obtained by subjecting the images to RGB image analysis that, even when the amount of p-Xy was 0.125, which was extremely small, a domain exhibiting reversibility occupied a large portion. From those results, it was found that, when the introduction amount of p-Xy was less than 0.5, and the carboxy group of PCDA could not be fully satisfied with the amino group of p-Xy, PDA-p-Xy was formed, and the formation of the composite of p-Xy with PCDA was more predominant than the formation of the composite of PAAm therewith.

Table 2. Introduction ratios of respective molecules of samples I to V in experiment of changing introduction distribution of *p-Xy* and PAAm

| Name of sample | Origin of amino group *p*-Xy:PAAm | *p*-Xy introduction amount (with respect to 1 mol of PCDA) | PAAm introduction amount (with respect to 1 mol of PCDA) |
|---|---|---|---|
| I. | 0:1 | 0 | 1 |
| II. | 0.25:0.75 | 0.125 | 0.75 |
| III. | 0.5:0.5 | 0.25 | 0.5 |
| IV. | 0.75:0.25 | 0.375 | 0.25 |
| V. | 1:0 | 0.5 | 0 |

(2-4) Observation of Composite with Electron Microscope

Next, secondary electron images of PDA-p-Xy and PAAm/PDA-p-Xy are shown in Fig. 24(A) to Fig. 24(D). A sample was produced by applying each of precursor solutions of PCDA-p-Xy and PAAm/PCDA-p-Xy to a silicon substrate by spray coating, and volatilizing the solution. PDA-p-Xy was in a state in which rectangular thin plate-like crystals each measuring about 1 μm long and about 5 μm wide were stacked, and PAAm/PDA-p-Xy was in a state in which rectangular thin plate-like crystals each measuring about 0.5 μm long and about 2 μm wide were aggregated. From the backscattered electron image, in PAAm/PDA-p-Xy, a white portion is observed around a plate-like portion (Fig. 24(D)). In the backscattered electron image, a white color represents an amorphous portion, and further, the white color is not observed in PDA-p-Xy (Fig. 24(C)). Accordingly, it is conceived that the white portion around the plate-like crystal in PAAm/PDA-p-Xy represents a location of PAAm.

It is conceived from the above-mentioned results that PAAm/PDA-p-Xy forms a "phase separation structure" of PDA-p-Xy, which has preferentially formed a composite, and PAAm present therearound (Fig. 25).

(3) Production of Composites using Various Low-molecular-weight Amines

(3-1) Production of Composites using Various Low-molecular-weight Amines

PAAm/PDA-guests were produced by using, as guests, other low-molecular-weight amines instead of p-Xy, and were evaluated. The low-molecular-weight amines used as guests in this case are shown below.

1,4-Bis(aminomethyl)cyclohexane ($c\text{-}C_6\text{-}(NH_2)_2$)

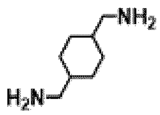

1,12-Diaminododecane ($C_{12}\text{-}(NH_2)_2$)

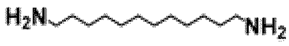

stearylamine ($C_{18}\text{-}NH_2$)

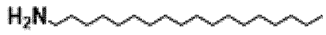

Low-molecular-weight amines used as guests

PAAm/PCDA-guests and PCDA-guests were each produced by using an ethanol solvent as in (1) of Test 3, and IR measurement results and XRD measurement results thereof are shown Fig. 26(A) and Fig. 26(B), respectively.

From the IR measurement results of Fig. 26(A), in any sample, a peak resulting from a carboxylic acid dimer disappeared and a peak resulting from a carboxylate ion appeared. Accordingly, it was found that the guests were intercalated between the PCDA layers. The peaks of the PCDA-guests and the PAAm/PCDA-guests were compared to each other, and the same degrees of peaks occurred at the same wavelengths in all the four guests. Accordingly, it was found that, in the PCDA-guests and the PAAm/PCDA-guests, the functional groups between the layers were in substantially the same state. In the XRD measurement results of Fig. 26(B), an increase in interlayer spacing d0 occurred in each sample. In addition, when the interlayer spacings d0 of the PCDA-guests and the PAAm/PCDA-guests were compared to each other, the interlayer spacings d0 became values extremely close to each other. It was found from those measurement results that molecules were newly introduced between the layers of PCDA in each of the PAAm/PCDA-guests and the PCDA-guests, and that changes in functional group states and increase widths of the interlayer spacings along with the introduction of the molecules corresponded to each other regardless of the presence or absence of PAAm.

(3-2) Temperature-responsive Color Change Behavior of Composite

Subsequently, the color change behavior at the time of the temperature control of each of the PAAm/PDA-guests was observed with an optical microscope. The respective samples were each produced by applying the solution onto a glass slide by spray coating, and the observation was performed by superimposing a clean sheet of paper on a glass back surface. Further, the color change behaviors of the PAAm/PDA-guests and the PDA-guests at a centimeter scale are shown in Fig. 27.

The red color change at from 80°C to 100°C and reversibility up to around 100°C of PAAm/PDA-$c\text{-}C_6\text{-}(CH_2NH_2)_2$ corresponded to those of PDA-$c\text{-}C_6\text{-}(CH_2NH_2)_2$. PAAm/PDA-$C_{12}\text{-}(NH_2)_2$ showed different color change behaviors at two sites (not shown). In one region, reversibility from red to blue was observed by a change from 100°C to 25°C, and it is conceived that the behavior is based on a PDA-$C_{12}\text{-}(NH_2)_2$ domain that shows a color change at 120°C and has reversibility. In the other region, a color change and irreversibility were observed in the range up to 100°C. The behavior is similar to that of a PDA-PAAm domain. Accordingly, there is a high possibility in that, in PAAm/PDA-$C_{12}\text{-}(NH_2)_2$, those two kinds of domains were separately produced. Also in Fig. 27, it was found that, in PAAm/PDA-$C_{12}\text{-}(NH_2)_2$, two kinds of domains were mixed. In PAAm/PDA-$C_{18}\text{-}NH_2$, a color change was completed by 80°C, and no reversibility was observed. The behavior substantially corresponds to PDA-$C_{18}\text{-}NH_2$ exhibiting a transitional color change at 85°C. Meanwhile, it appears from Fig. 27 that PDA-PAAm is mixed.

From the above-mentioned results, when $c\text{-}C_6\text{-}(CH_2NH_2)_2$ and $C_{18}\text{-}NH_2$ were used as the guests, the PCDA-guests were preferentially formed as composites. It is conceived that the phase separation structure of Fig. 25 is formed in each of the PAAm/PDA-guests using those composites in the same manner as in PAAm/PDA-p-Xy. Meanwhile, it was found that, when PDA-$C_{12}\text{-}(NH_2)_2$ was used as the guest, both of the PDA-guest and PDA-PAAm were formed. Accordingly, in order to produce an ideal single PDA domain for forming a PAAm/PDA-guest phase separation structure that exhibits the temperature-responsive color change behavior of the PDA-guest, a guest that easily causes composite formation and aggregation of the PCDA-guest as typified by p-Xy is preferably selected.

(4) Optimization of Coating Method

(4-1) Selection of Coating Method

The amount of PCDA was set to 1, the amount of p-Xy was set to 0.5 (molar ratio), and the amount of PAAm was set to 0 mol and 1.3 mol, and spray coating, dip coating, and drop casting were investigated as coating methods.

In the spray coating, 10 mL of a PAAm/PCDA-p-Xy precursor solution was sprayed from a spray container to a 2 cm×2 cm substrate at 45° from diagonally above. The number of times of the spraying was set to one. In the dip coating, a substrate was immersed in 20 mL of the PAAm/PCDA-p-Xy precursor solution prepared in a beaker for 10 seconds, and was then lifted. In the drop casting, a substrate was left to stand still in 10 mL of the PAAm/PCDA-p-Xy precursor solution

prepared in a petri dish, and was then left to stand as it was for about a day and a half so that the solvent was volatilized. A silk cloth substrate and a filter paper substrate were used as the substrates.

In each of the substrates, the coating became most uniform in the spray coating when observed at a centimeter scale. The drop casting requires a long period of time for solvent volatilization, and hence it is conceived that crystals aggregated at various points by an intermolecular force during that period, resulting in a nonuniform state. The dip coating is not suitable as the coating method because the amount of the solution that can adhere to the substrate is small. It is conceived that the spray coating is suitable for uniform coating from the viewpoints that fine liquid droplets are uniformly released into a space, and that a certain amount of the solvent volatilizes at the time of spraying.

In addition, also in the spray coating in which the most uniform coating property was observed, nonuniformity was observed when the amount of PAAm was 0 mol. Accordingly, an improvement in coating property at a centimeter scale along with the introduction of PAAm was able to be observed.

In addition, in secondary electron image (SEM) observation, in each of the silk cloth substrate and the filter paper substrate, when the introduction amount of PAAm was 0, a state in which a plate-like crystal of PDA having a size of about 10 μm was aligned in a fibrous manner was established, and when the introduction amount of PAAm was 1.3, a state in which a spherical crystal having a size of from about 1 μm to about 2 μm was entangled with the fiber was established (data not shown). Coating that was less liable to be peeled off and was physically stable was achieved by the introduction of PAAm.

(4-2) Increase in Area of Device

Subsequently, the method of spraying was improved, and a greater variety of substrates (about 3 cm×3 cm, polyurethane, hard polyethylene, polypropylene, a silk cloth, a polyethylene fiber, and polystyrene) were subjected to PAAm/PDA-p-Xy coating. Specifically, an operation of spraying the substrate with a spray three times, and then drying the substrate with a cold air dryer was repeated about six or seven times. The obtained PCDA/guest composite was subjected to solid-state photopolymerization by irradiation with UV light. A UV-LED lamp (CCS Inc., HLDL-120X30UV265-PSC, 265 nm) or a hand lamp (AS ONE, Handy UV Lamp SLUV-6, 254 nm) was used as a light source.

It was found from the above-mentioned optimization that, when a PCDA-p-Xy-PAAm precursor solution obtained by introducing 0.5 of p-Xy and 1 to 1.3 of PAAm with respect to 1 of PCDA (molar ratio) was applied by spray coating by the above-mentioned method, a uniform increase in area of any substrate was able to be performed. A PAAm/PDA-p-Xy silk cloth substrate was produced under the same conditions, color behavior through temperature increase every 5°C from 25°C to 115°C and cooling to 20°C every 10°C was observed (Fig. 30). In addition, a relationship between $\Delta x_{st}$ obtained by standardizing a red intensity change Δx determined by image analysis and a temperature was shown in the graph of Fig. 31.

It was shown from the image of Fig. 30 and the graph and the image of Fig. 31 that the produced PAAm/PDA-p-Xy fabric device showed a color change from about 80°C and reversibility up to around 110°C, and uniform coating was obtained.

Explanation of Reference Numerals

10 temperature imaging device, 11···base material,
12 composite.

## Claims

1. A temperature imaging device comprising:

   a base material; and
   a composite having polyallylamine introduced between layers of poly-10,12-pentacosadiynoic acid,
   the composite being incorporated into the base material or caused to adhere onto the base material.

2. The temperature imaging device according to claim 1, wherein the temperature imaging device has an initial value of a red intensity "x" before heating of 0.280 or more and a value of a change amount Δx of the red intensity "x" when a color change is caused by heating to 100°C of 0.025 or more.

3. A temperature imaging device comprising:

a base material; and
a composite of poly-10,12-pentacosadiynoic acid, polyallylamine, and an amine compound,
the composite being incorporated into the base material or caused to adhere onto the base material,
wherein at least part of the polyallylamine and/or the amine compound is introduced between layers of the poly-10,12-pentacosadiynoic acid.

**4.** The temperature imaging device according to claim 1 or 3, wherein a plurality of droplets of the composite adhere to the base material in a substantially spherical shape under a dispersed state.

**5.** The temperature imaging device according to claim 1 or 3,

wherein the base material has a sheet shape, and
wherein the temperature imaging device is a sheet-shaped device.

**6.** The temperature imaging device according to claim 1 or 3, wherein the base material includes a woven fabric or a nonwoven fabric.

**7.** The temperature imaging device according to claim 1,
wherein the base material is a sheet-shaped base material having a length of 100 mm or more and a width of 100 μm or more.

**8.** The temperature imaging device according to claim 1,
wherein the temperature imaging device has a color change start temperature lower than 30°C and a color change end temperature higher than 80°C.

**9.** A method of estimating a specific heat of a liquid, the method comprising:

heating a container containing the liquid, the container having been sealed, to a certain temperature;
measuring a color change of the temperature imaging device of claim 1 or 3 attached to the container;
calculating a specific heat equivalent value of the liquid from a time period from a start of heating until the color change stops and a change amount Δx of a red intensity "x" of the temperature imaging device from the start of the heating until the color change stops; and
substituting the specific heat equivalent value of the liquid into a functional formula for a specific heat and a specific heat equivalent value to calculate the specific heat of the liquid.

**10.** A method of examining a temperature change of a test site, the method comprising:

arranging the temperature imaging device of claim 1 or 3 on the test site; and
detecting a color change of the temperature imaging device arranged on the test site after heating.

**11.** The method according to claim 10, wherein the detecting the color change includes detecting, after heating with an energy device for a surgery, the color change of the temperature imaging device arranged on an affected part subjected to heating treatment or a vicinity thereof.

**12.** An energy device for a surgery comprising a coating caused to adhere to the energy device, the coating including a composite having polyallylamine introduced between layers of poly-10,12-pentacosadiynoic acid.

**13.** An energy device for a surgery comprising a coating caused to adhere to the energy device, the coating including a composite containing poly-10,12-pentacosadiynoic acid, polyallylamine, and an amine compound, the composite having at least part of the polyallylamine and/or the amine compound introduced between layers of the poly-10,12-pentacosadiynoic acid.

**14.** A method of producing a temperature imaging device, the method comprising:

applying, to a base material, a composition containing 10,12-pentacosadiynoic acid, polyallylamine having a unit represented by the following general formula (1), and a solvent by spray coating;
removing the solvent; and
polymerizing the 10,12-pentacosadiynoic acid to form a composite having the polyallylamine introduced between

layers of poly-10,12-pentacosadiynoic acid:

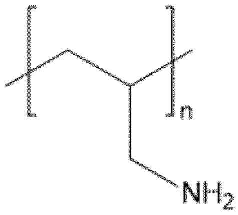

(1)

where "n" represents an integer.

**15.** A method of producing a temperature imaging device, the method comprising:

applying, to a base material, a composition containing 10,12-pentacosadiynoic acid, polyallylamine having a unit represented by the following general formula (1), an amine compound, and a solvent by spray coating;
removing the solvent; and
polymerizing the 10,12-pentacosadiynoic acid to form a composite having at least part of the polyallylamine and/or the amine compound introduced between layers of poly-10,12-pentacosadiynoic acid:

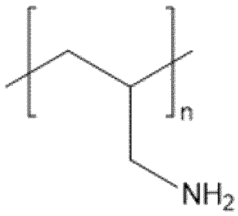

(1)

where "n" represents an integer.

**16.** The method according to claim 14 or 15,
wherein the forming the composite includes forming the composite so that the composite adheres to the base material in a substantially spherical shape.

Fig. 1

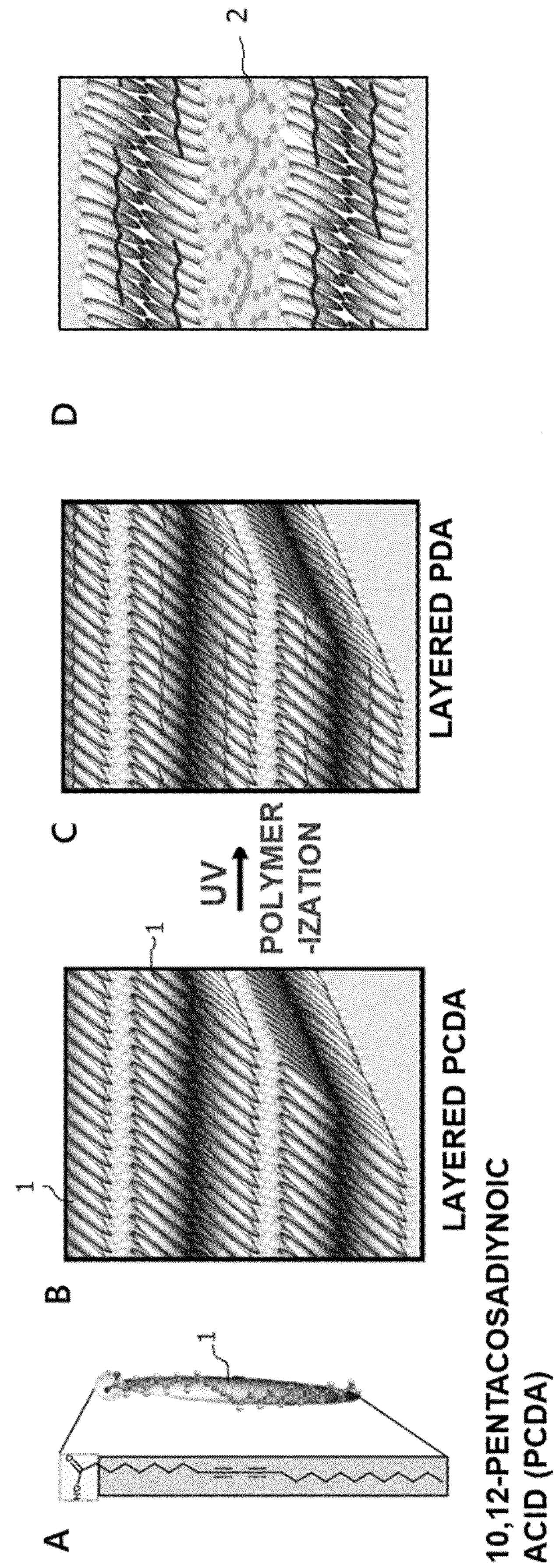
A
10,12-PENTACOSADIYNOIC
ACID (PCDA)
1
B
LAYERED PCDA
1
1
UV
POLYMER
-IZATION
C
LAYERED PDA
D
2

Fig. 2

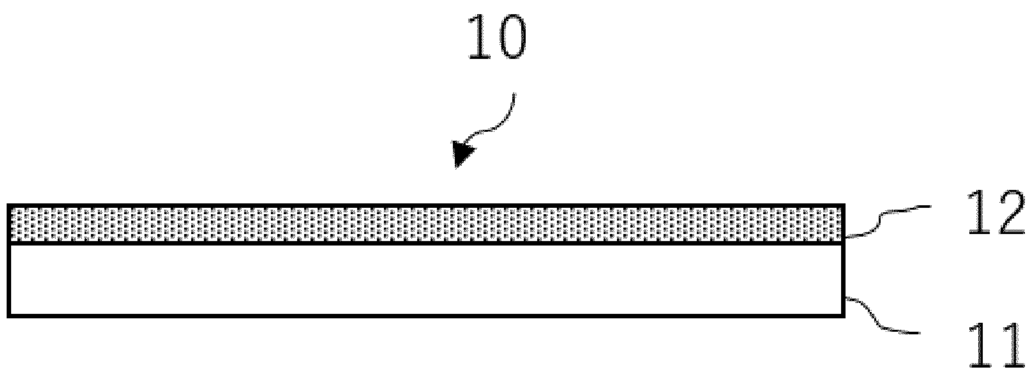

Fig. 3

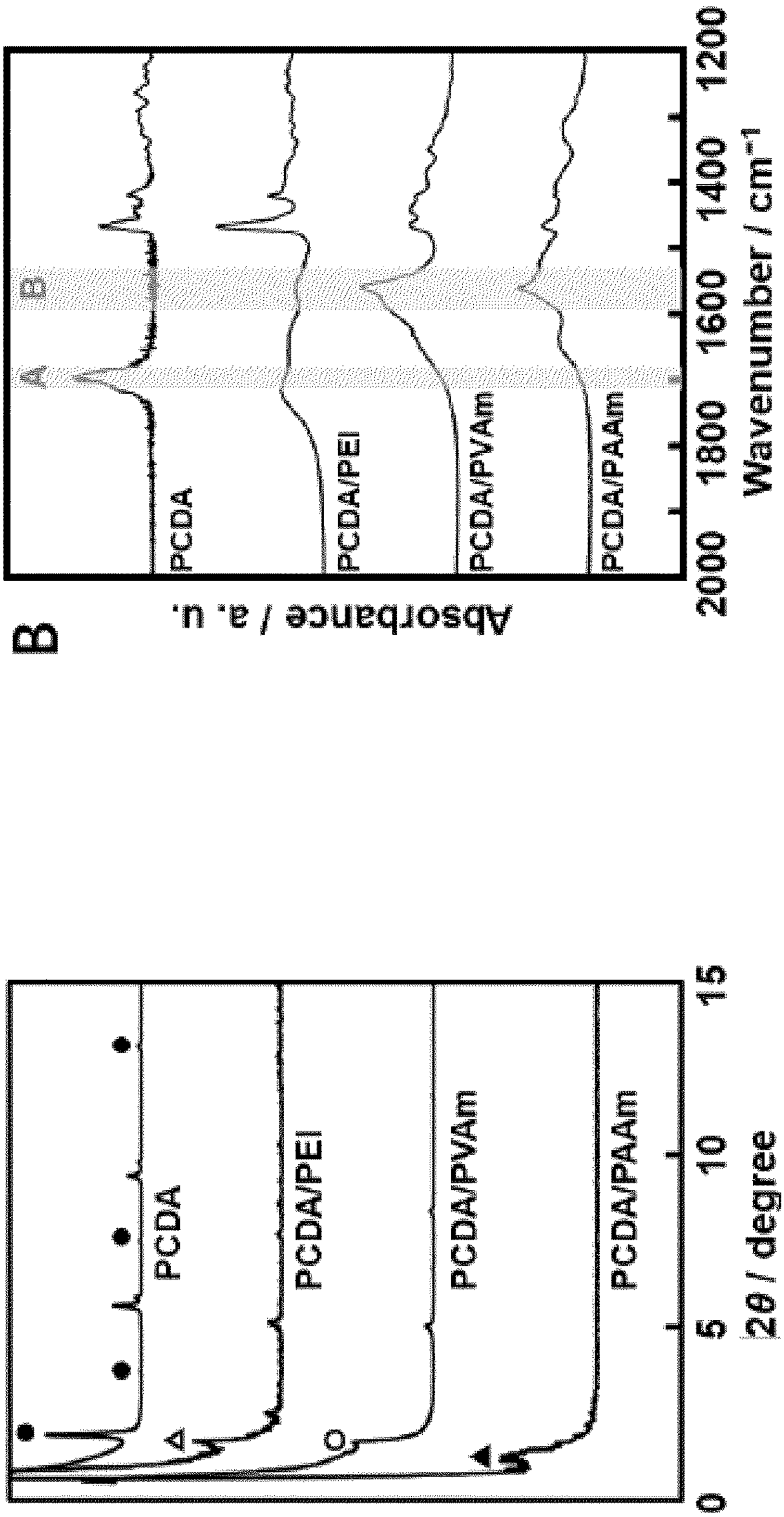
A
Intensity / a. u.
PCDA
PCDA/PEI
PCDA/PVAm
PCDA/PAAm
2θ / degree
0
5
10
15
B
Absorbance / a. u.
A
B
PCDA
PCDA/PEI
PCDA/PVAm
PCDA/PAAm
Wavenumber / cm⁻¹
2000
1800
1600
1400
1200

Fig. 4

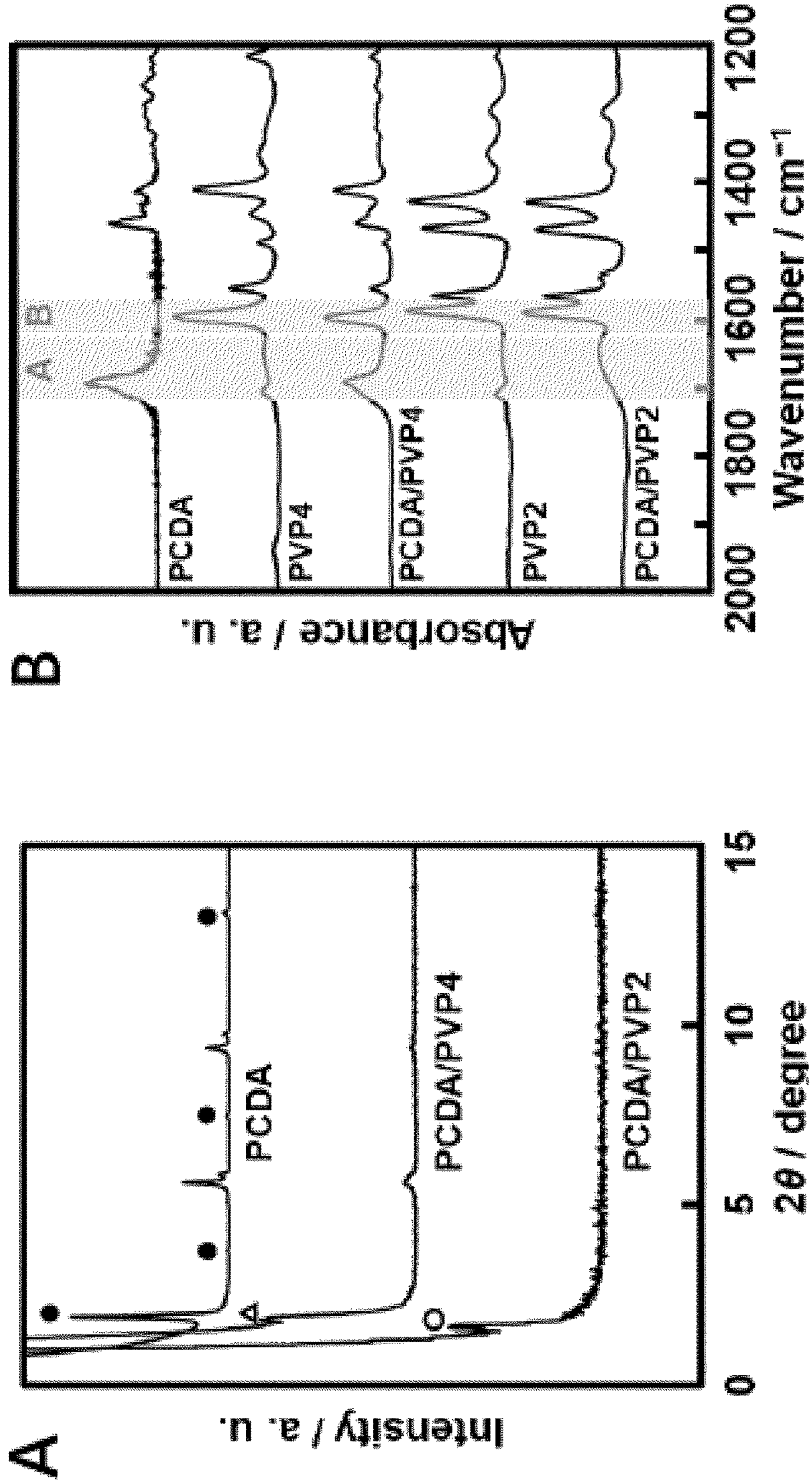
A
Intensity / a. u.
2θ / degree
0
5
10
15
PCDA
PCDA/PVP4
PCDA/PVP2
B
Absorbance / a. u.
Wavenumber / cm⁻¹
2000
1800
1600
1400
1200
PCDA
PVP4
PCDA/PVP4
PVP2
PCDA/PVP2
A
B

Fig. 5

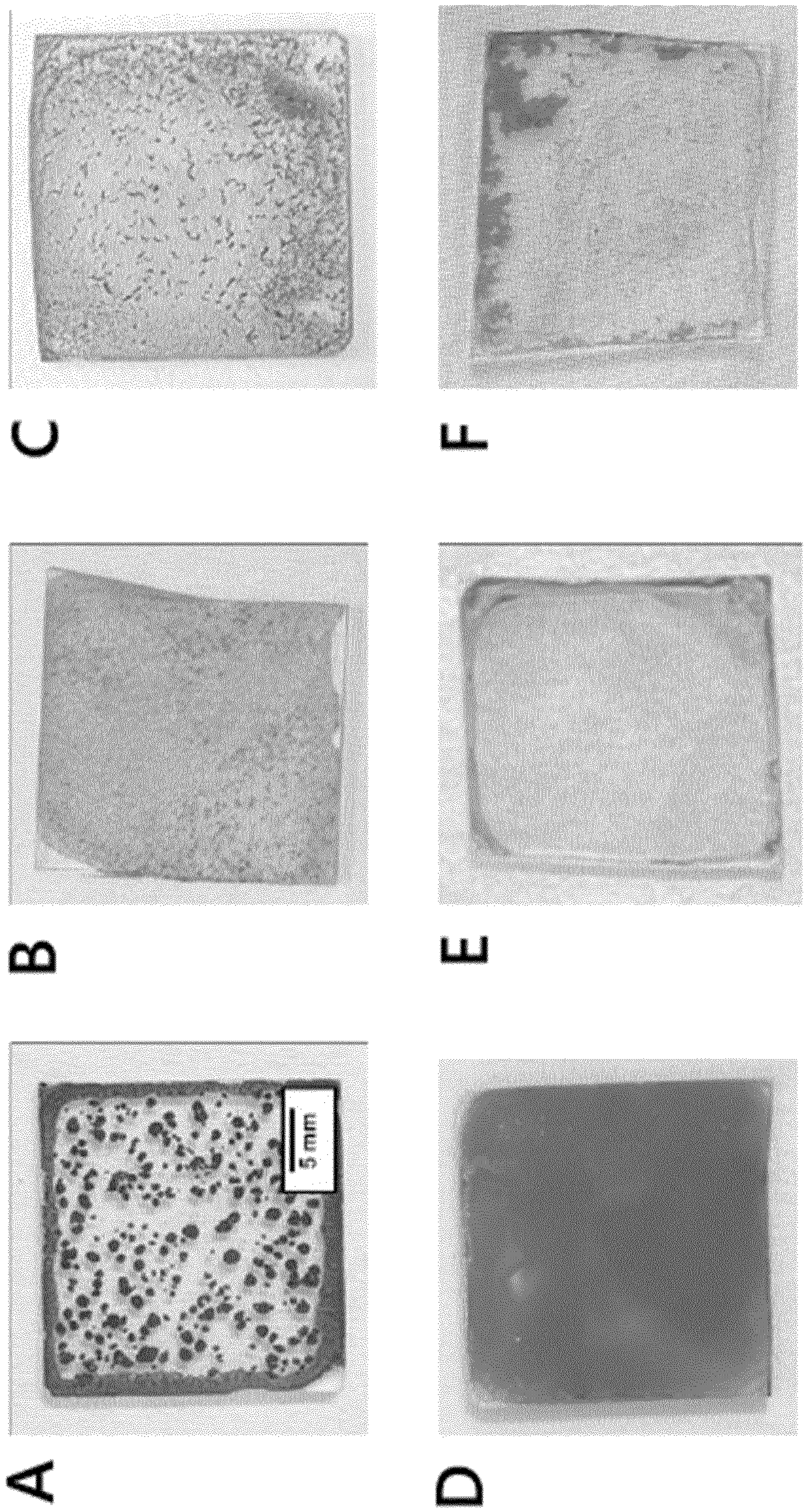

Fig. 6

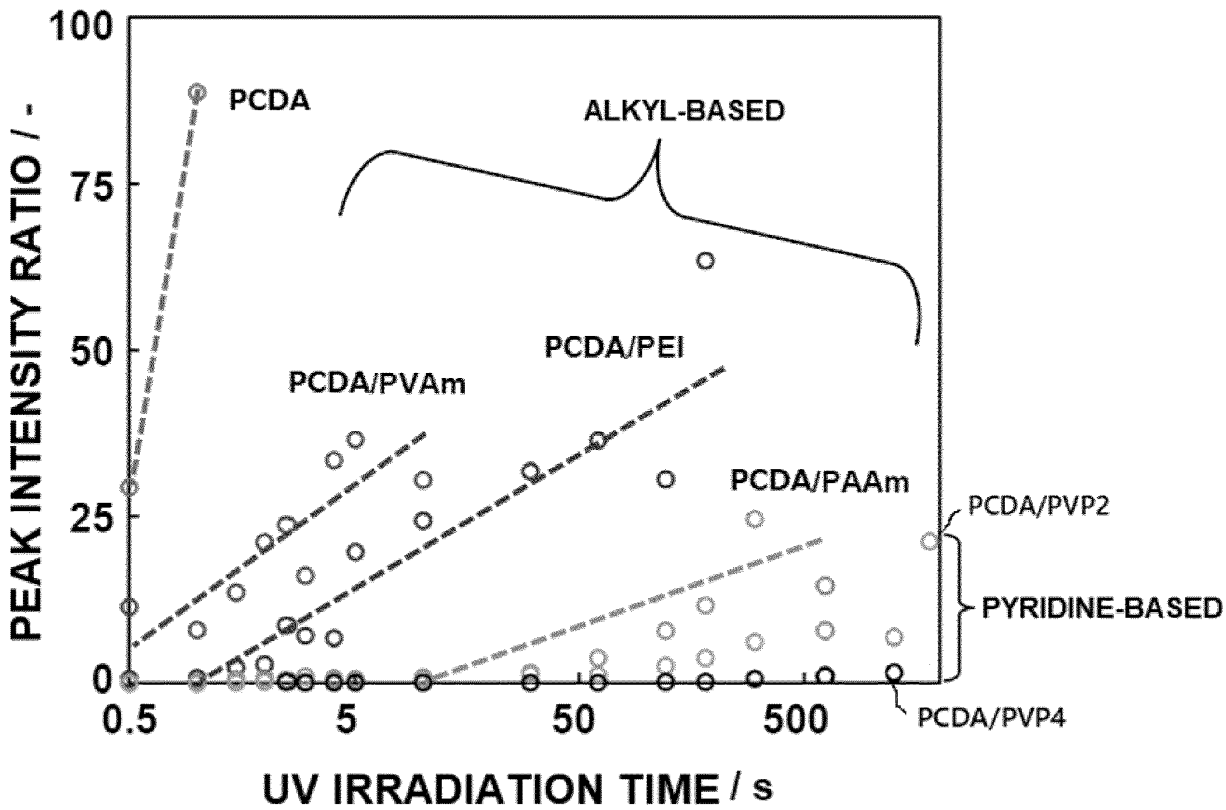
PEAK INTENSITY RATIO / -
100
75
50
25
0
0.5
5
50
500
UV IRRADIATION TIME / s
PCDA
ALKYL-BASED
PCDA/PEI
PCDA/PVAm
PCDA/PAAm
PCDA/PVP2
PYRIDINE-BASED
PCDA/PVP4

Fig. 7

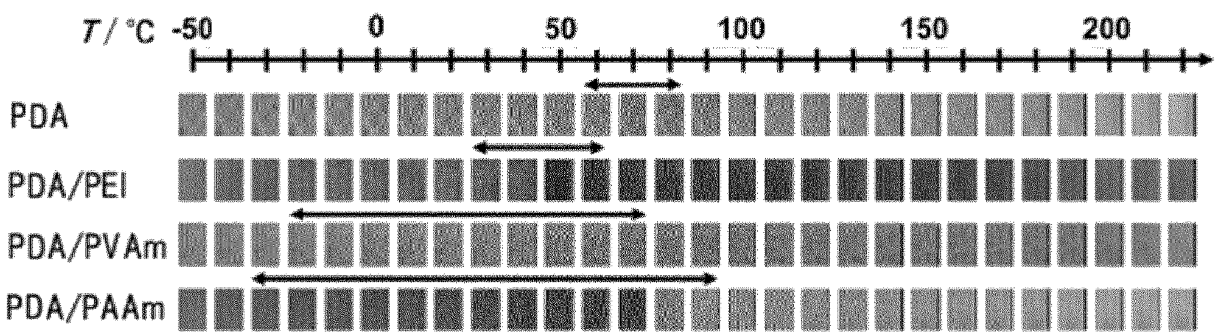
T / °C
-50
0
50
100
150
200
PDA
PDA/PEI
PDA/PVAm
PDA/PAAm

Fig. 8

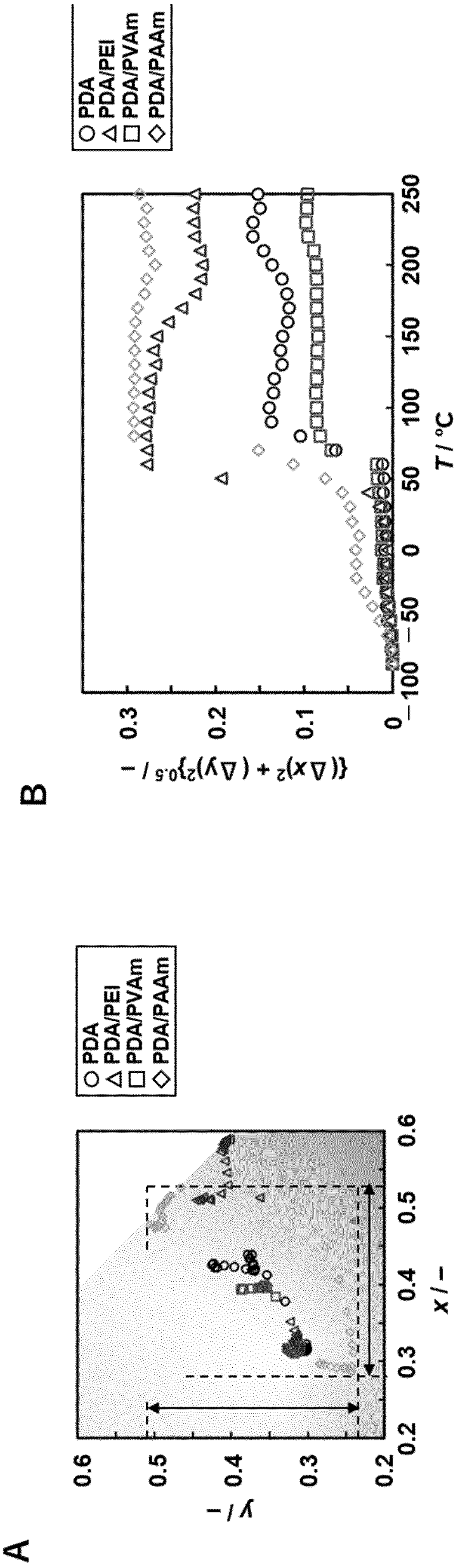
A
PDA
PDA/PEI
PDA/PVAm
PDA/PAAm
y / –
x / –
B
PDA
PDA/PEI
PDA/PVAm
PDA/PAAm
{(Δx)2 + (Δy)2}0.5 / –
T / °C

Fig. 9

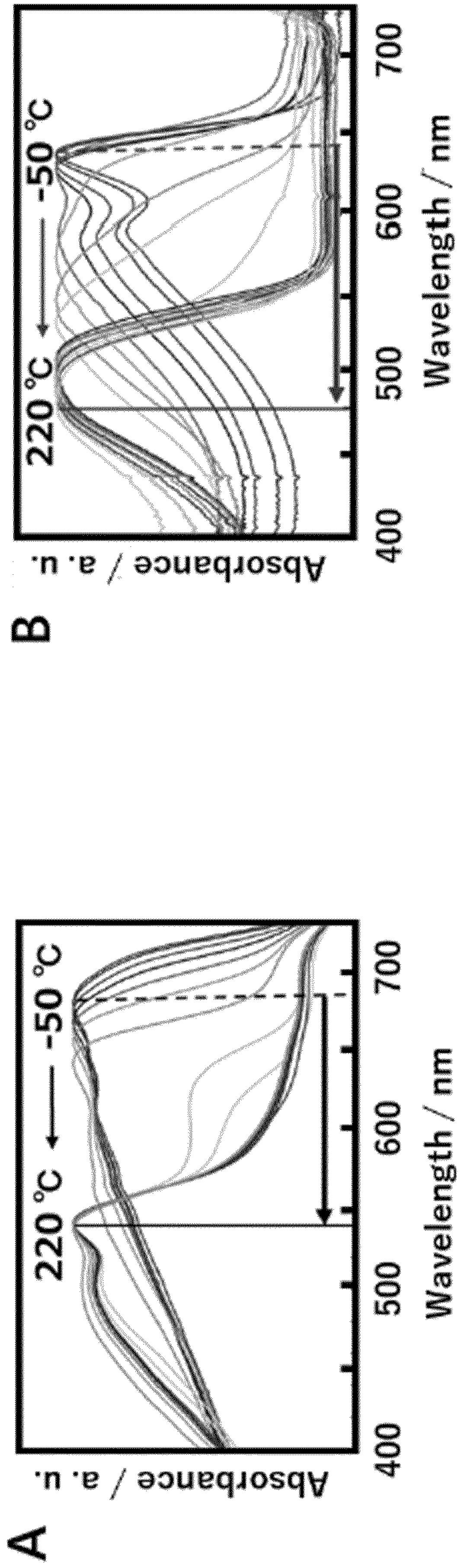
A
Absorbance / a. u.
220 °C ← -50 °C
Wavelength / nm
400
500
600
700
B
Absorbance / a. u.
220 °C ← -50 °C
Wavelength / nm
400
500
600
700

Fig. 10

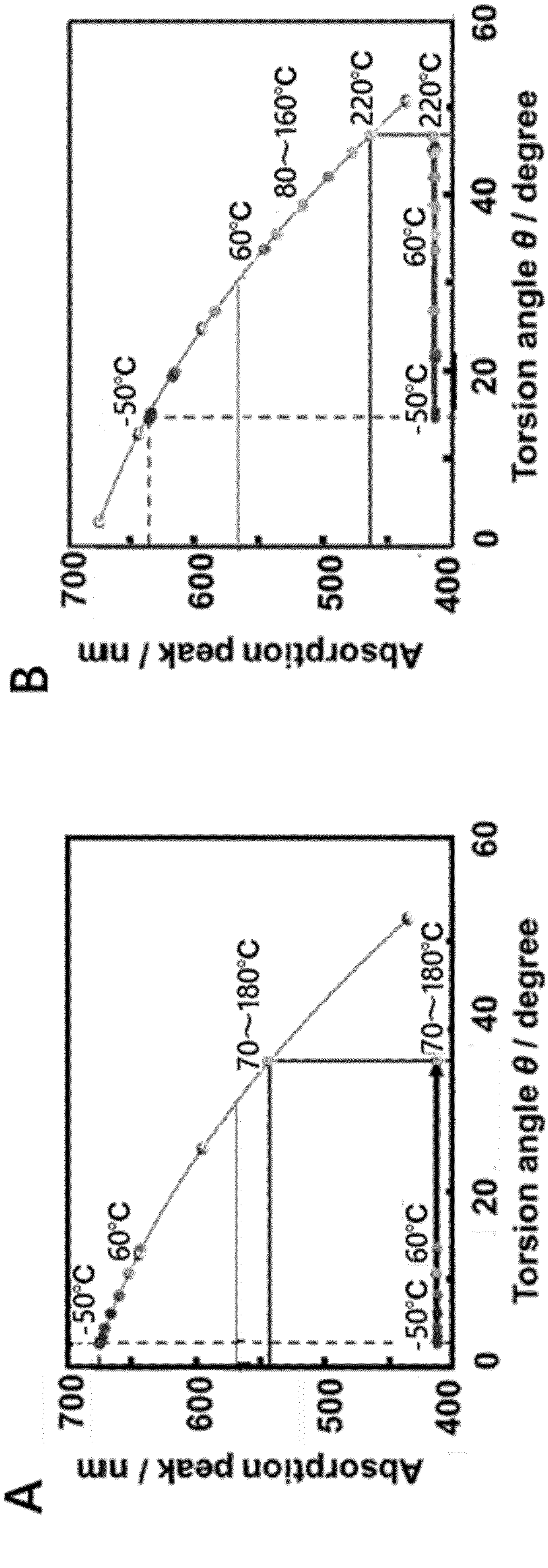
A
Absorption peak / nm
700
600
500
400
0
20
40
60
Torsion angle θ / degree
-50°C
60°C
70~180°C
-50°C
60°C
70~180°C
B
Absorption peak / nm
700
600
500
400
0
20
40
60
Torsion angle θ / degree
-50°C
60°C
80~160°C
220°C
-50°C
60°C
220°C

Fig. 11

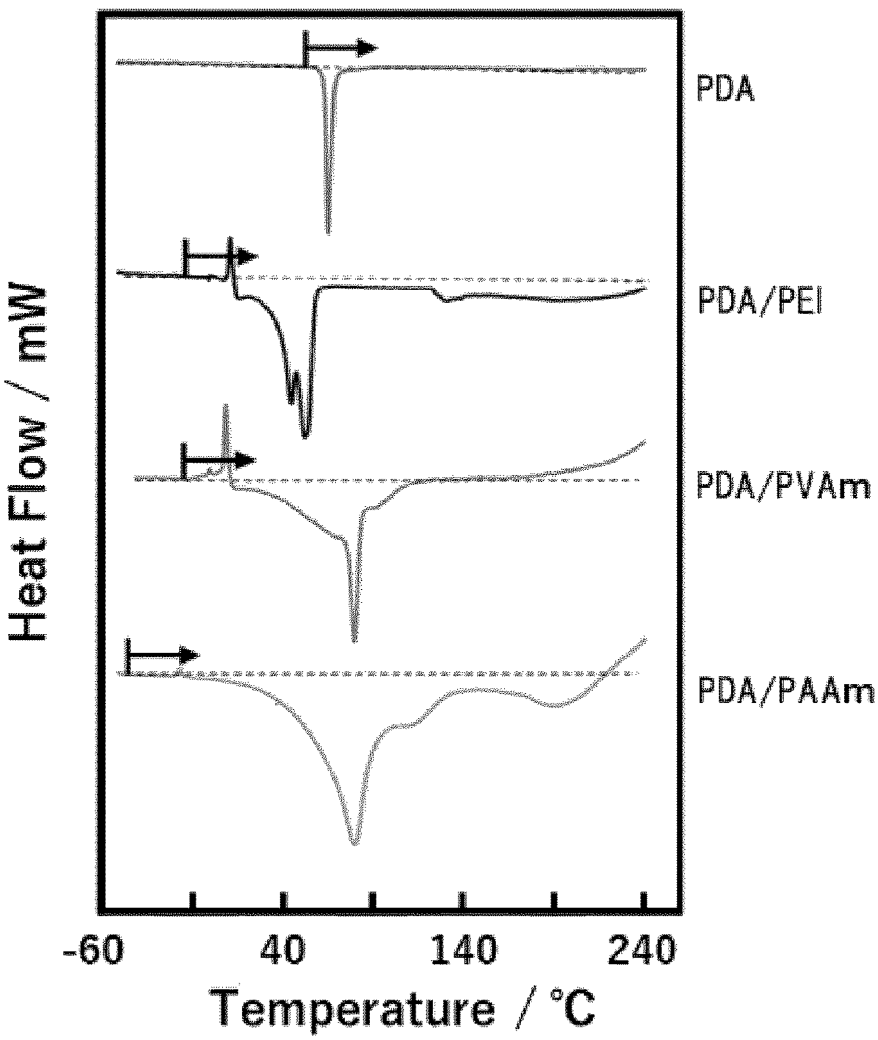
PDA
PDA/PEI
PDA/PVAm
PDA/PAAm
Heat Flow / mW
-60
40
140
240
Temperature / °C

Fig. 12

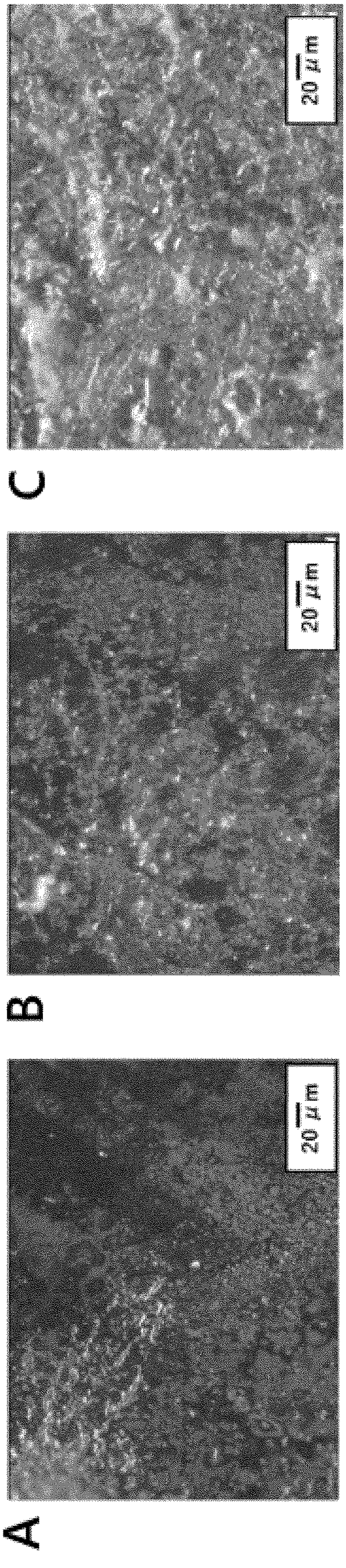

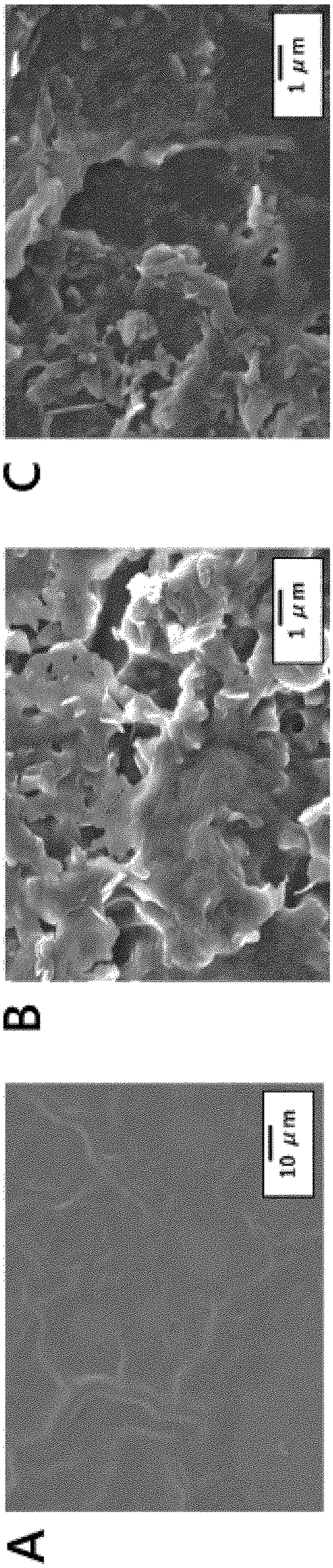


Fig. 13

Fig. 14

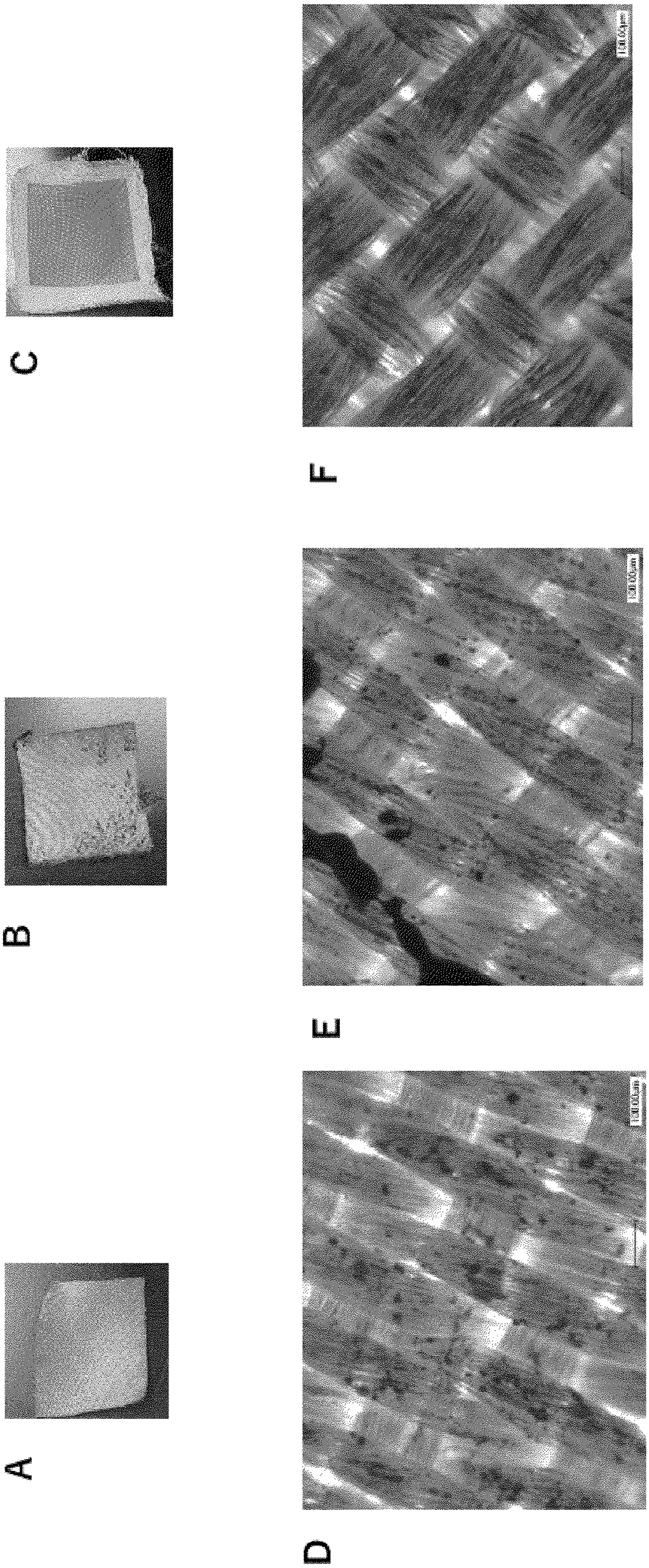

Fig. 15

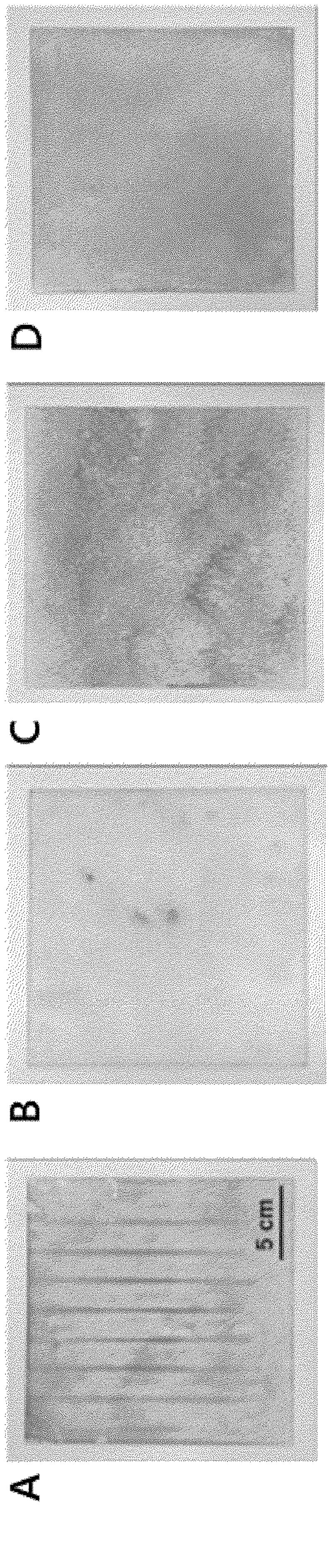

Fig. 16

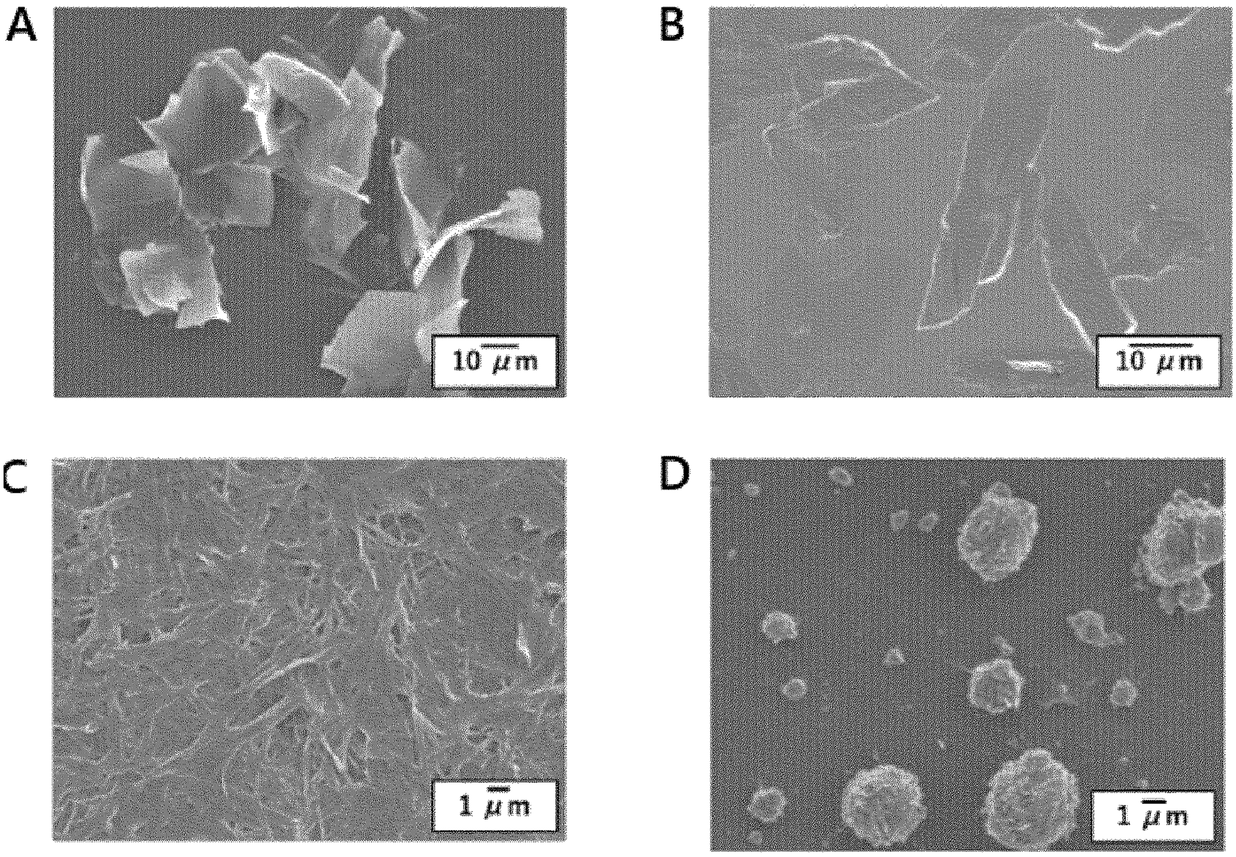
A
10 μm
B
10 μm
C
1 μm
D
1 μm

Fig. 17

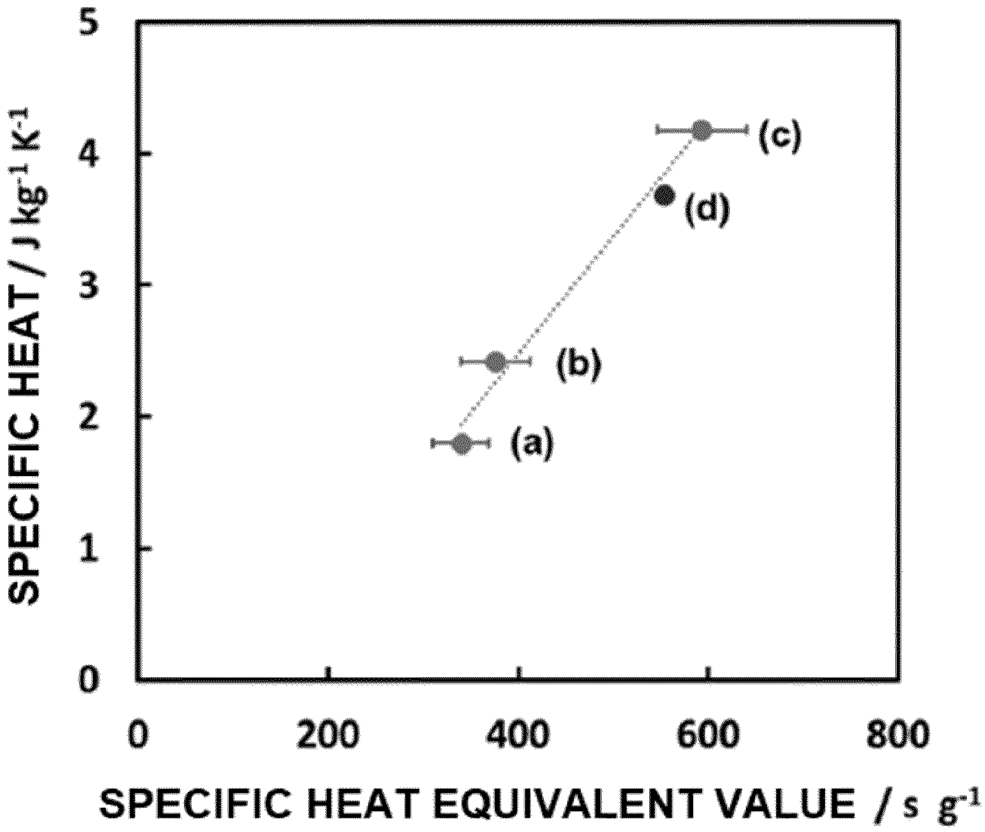
SPECIFIC HEAT / J kg-1 K-1
SPECIFIC HEAT EQUIVALENT VALUE / s g-1
0
1
2
3
4
5
0
200
400
600
800
(a)
(b)
(c)
(d)

Fig. 18

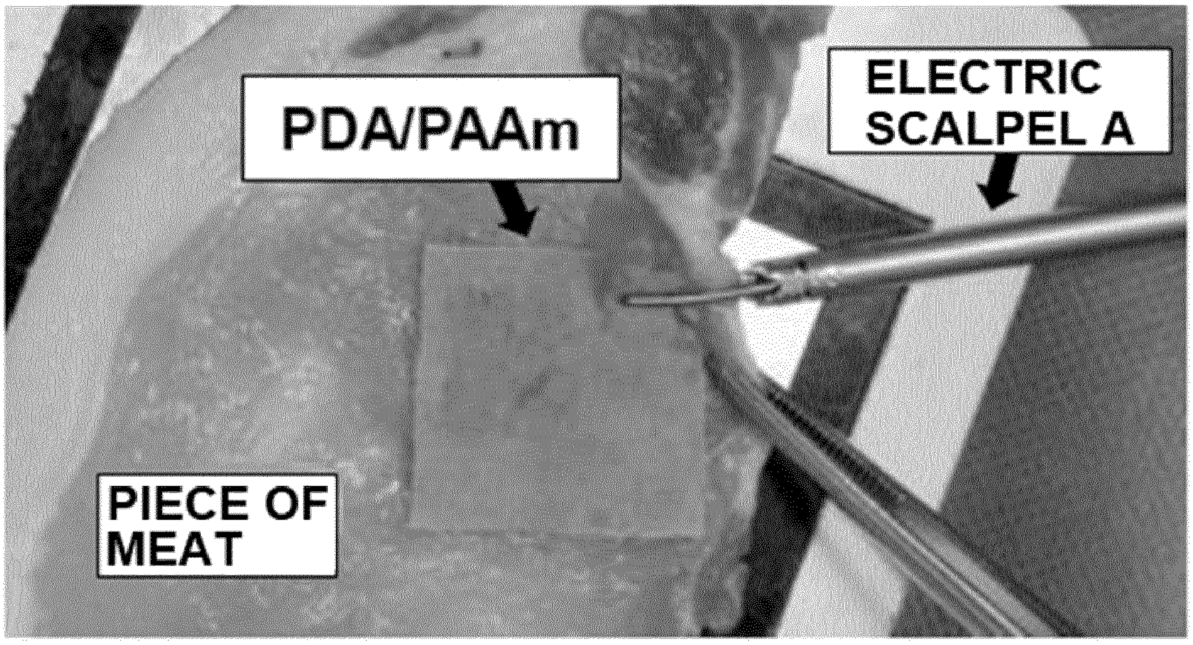

Fig. 19

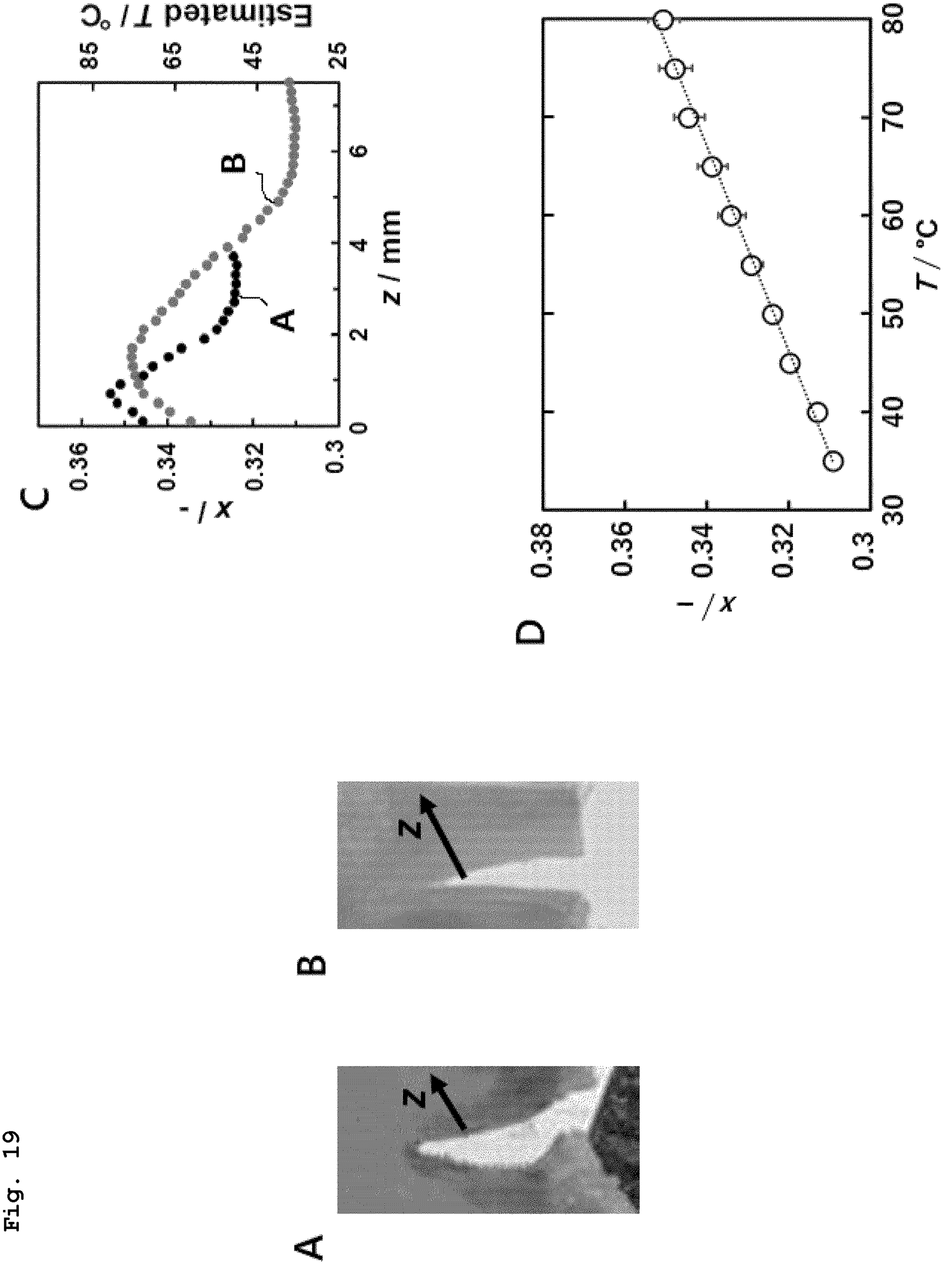
A
z
B
z
C
x / -
0.36
0.34
0.32
0.3
0
2
4
6
z / mm
Estimated T / °C
85
65
45
25
A
B
D
x / -
0.38
0.36
0.34
0.32
0.3
30
40
50
60
70
80
T / °C

Fig. 20

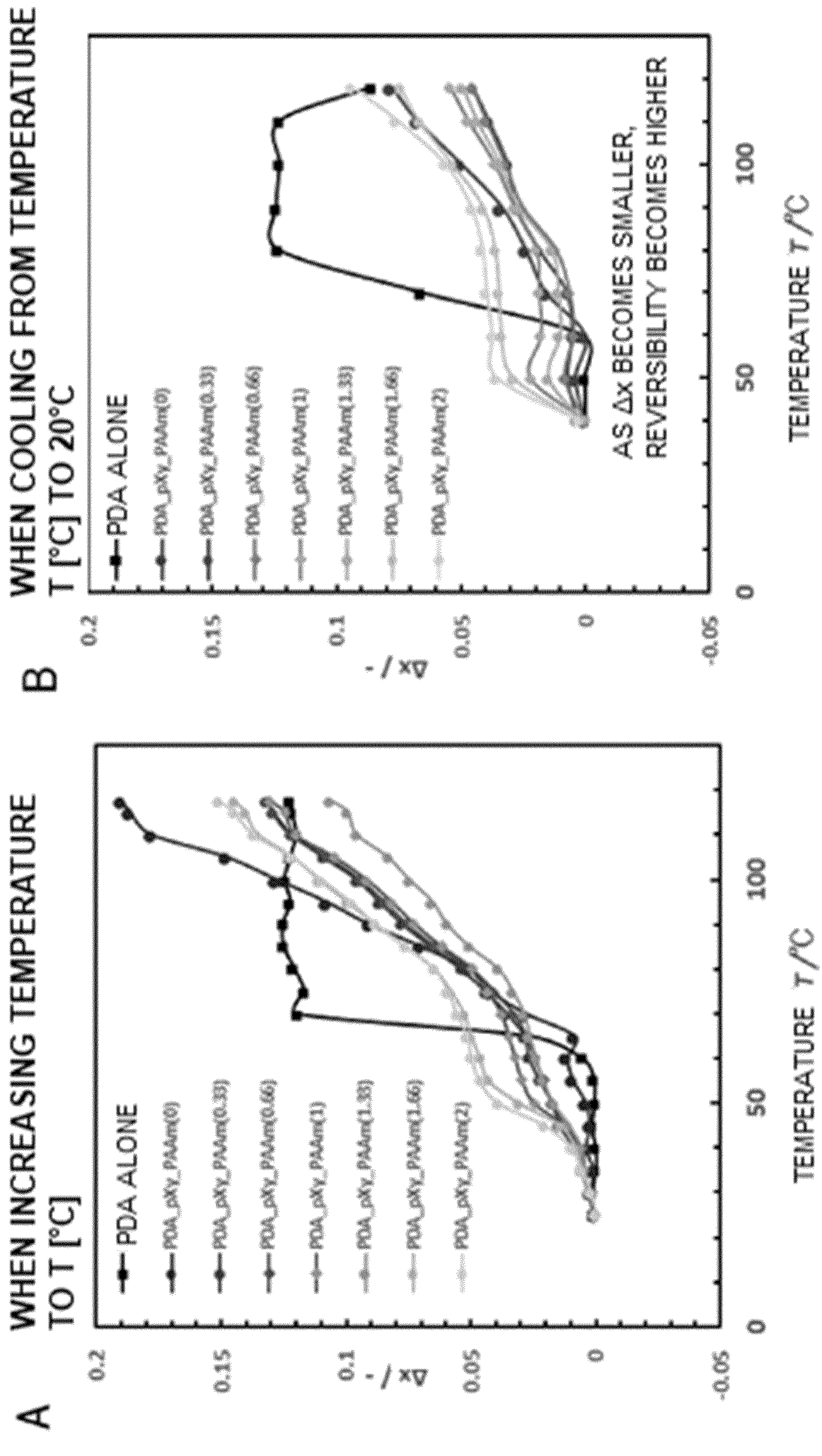
A
WHEN INCREASING TEMPERATURE TO T [°C]
PDA ALONE
PDA_pXy_PAAm(0)
PDA_pXy_PAAm(0.33)
PDA_pXy_PAAm(0.66)
PDA_pXy_PAAm(1)
PDA_pXy_PAAm(1.33)
PDA_pXy_PAAm(1.66)
PDA_pXy_PAAm(2)
Δx / -
0.2
0.15
0.1
0.05
0
-0.05
0
50
100
TEMPERATURE T/°C
B
WHEN COOLING FROM TEMPERATURE T [°C] TO 20°C
PDA ALONE
PDA_pXy_PAAm(0)
PDA_pXy_PAAm(0.33)
PDA_pXy_PAAm(0.66)
PDA_pXy_PAAm(1)
PDA_pXy_PAAm(1.33)
PDA_pXy_PAAm(1.66)
PDA_pXy_PAAm(2)
AS Δx BECOMES SMALLER, REVERSIBILITY BECOMES HIGHER
Δx / -
0.2
0.15
0.1
0.05
0
-0.05
0
50
100
TEMPERATURE T/°C

Fig. 21

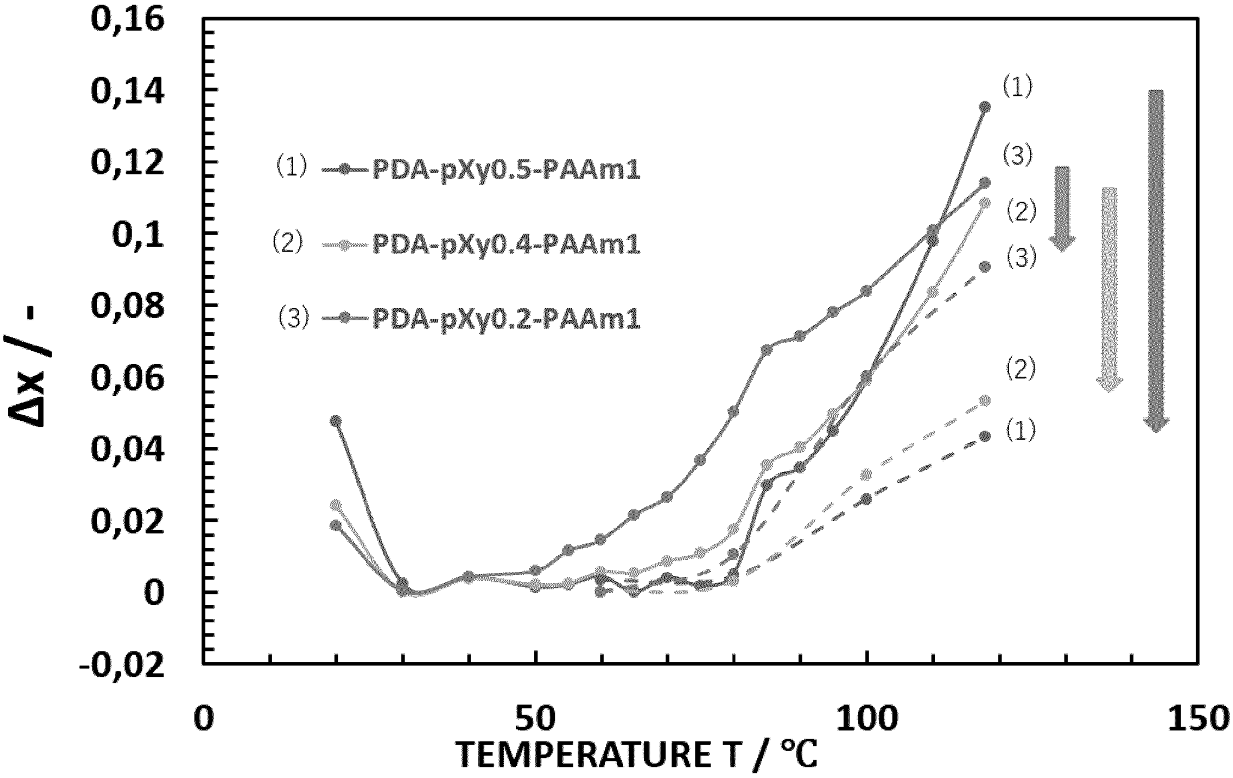
Δx / -
TEMPERATURE T / ℃
(1) PDA-pXy0.5-PAAm1
(2) PDA-pXy0.4-PAAm1
(3) PDA-pXy0.2-PAAm1
(1)
(3)
(2)
(3)
(2)
(1)
0,16
0,14
0,12
0,1
0,08
0,06
0,04
0,02
0
-0,02
0
50
100
150

Fig. 22

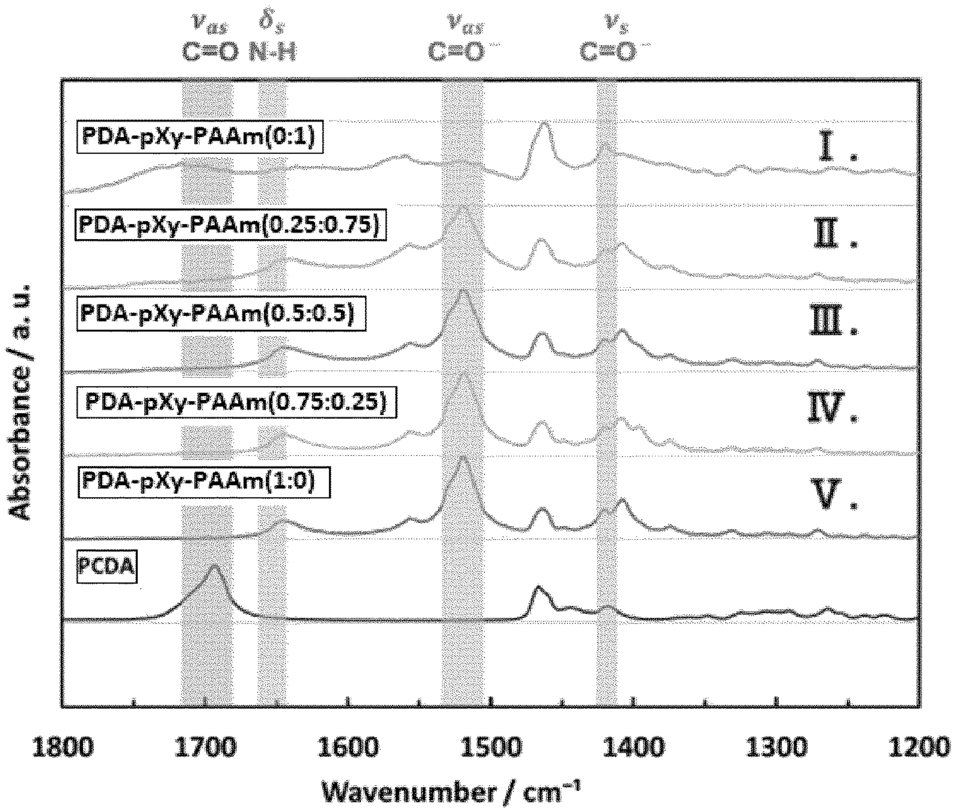
$\nu_{as}$ C=O
$\delta_s$ N-H
$\nu_{as}$ C=O⁻
$\nu_s$ C=O⁻
PDA-pXy-PAAm(0:1)
I.
PDA-pXy-PAAm(0.25:0.75)
II.
PDA-pXy-PAAm(0.5:0.5)
III.
PDA-pXy-PAAm(0.75:0.25)
IV.
PDA-pXy-PAAm(1:0)
V.
PCDA
Absorbance / a. u.
1800
1700
1600
1500
1400
1300
1200
Wavenumber / cm⁻¹

Fig. 23

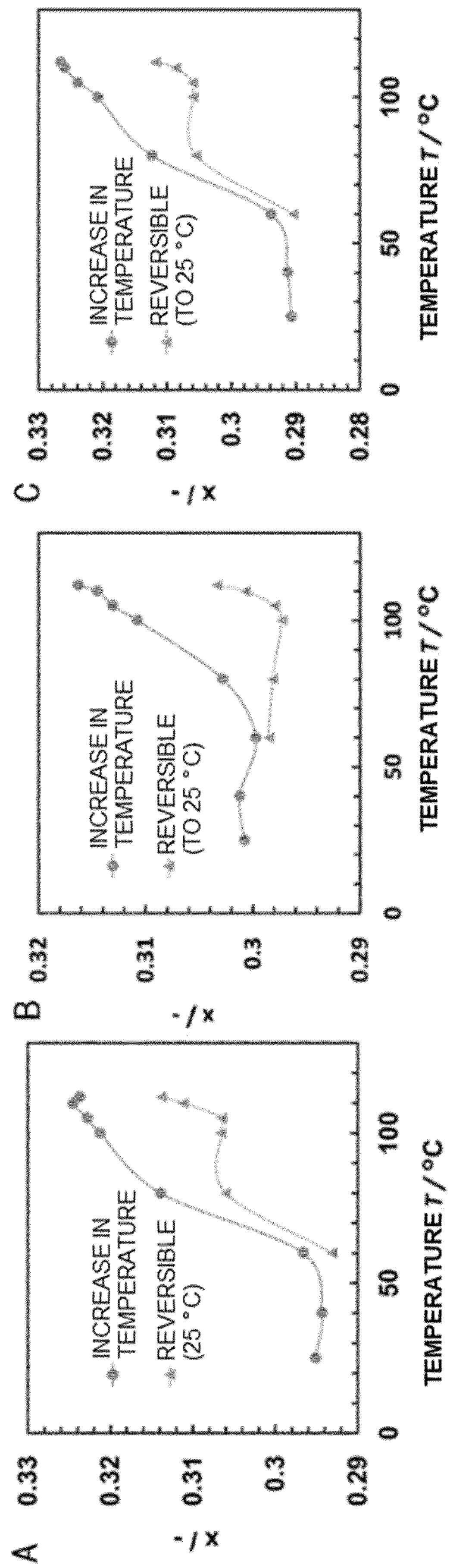
A
B
C
x / -
0.33
0.32
0.31
0.3
0.29
0.28
INCREASE IN TEMPERATURE
REVERSIBLE (25 °C)
REVERSIBLE (TO 25 °C)
0
50
100
TEMPERATURE T / °C

Fig. 24

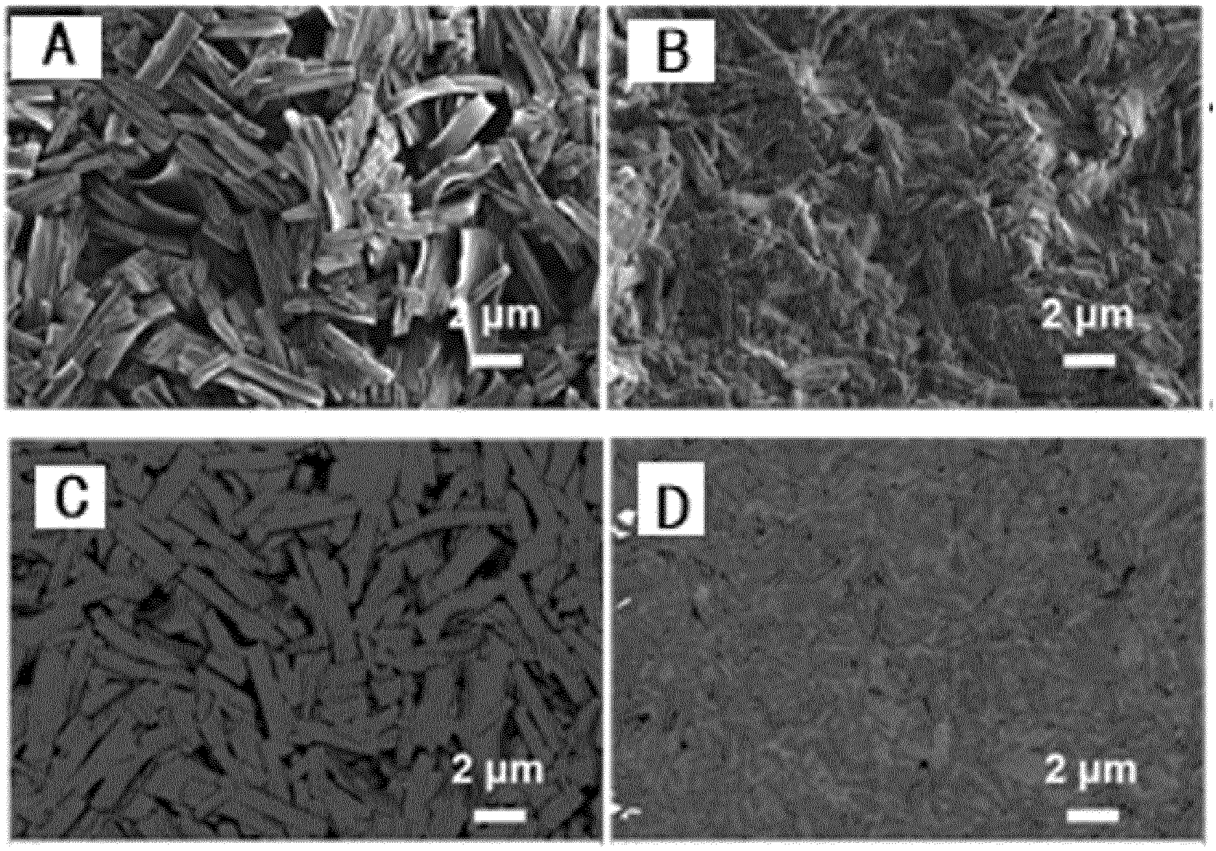

Fig. 25

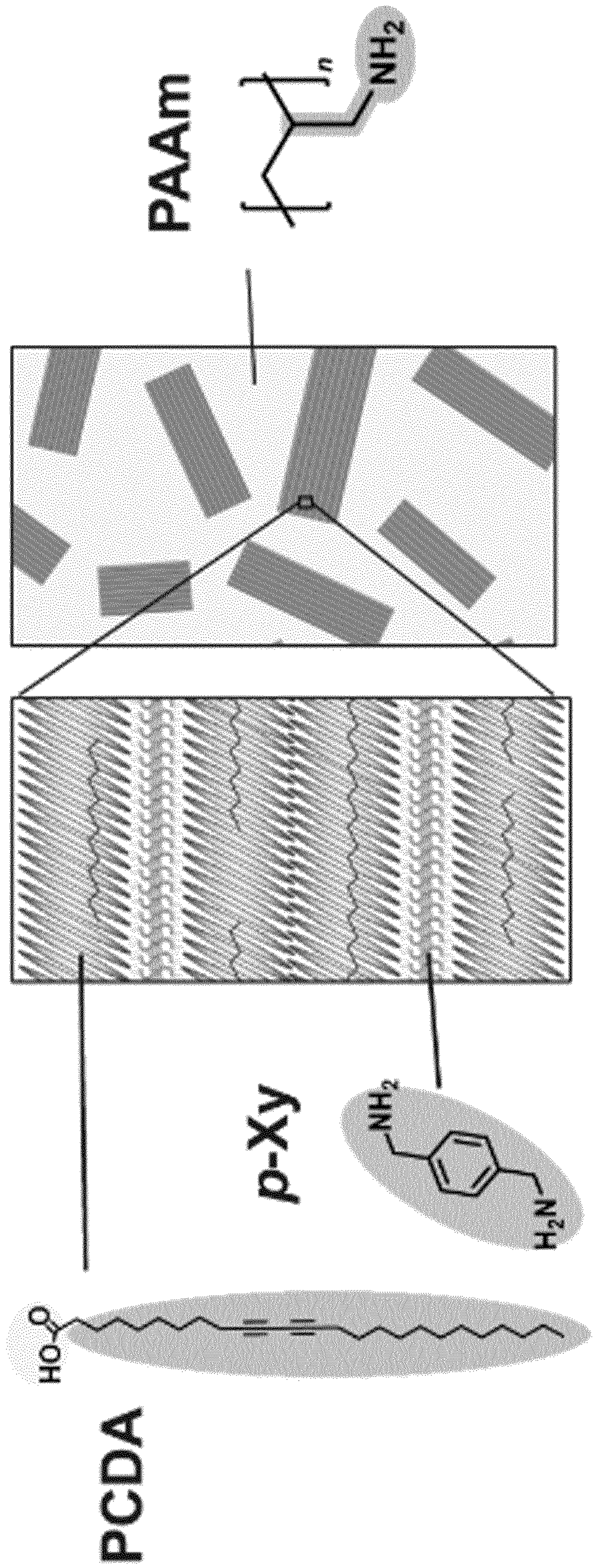

PCDA

*p*-Xy

PAAm

Fig. 26

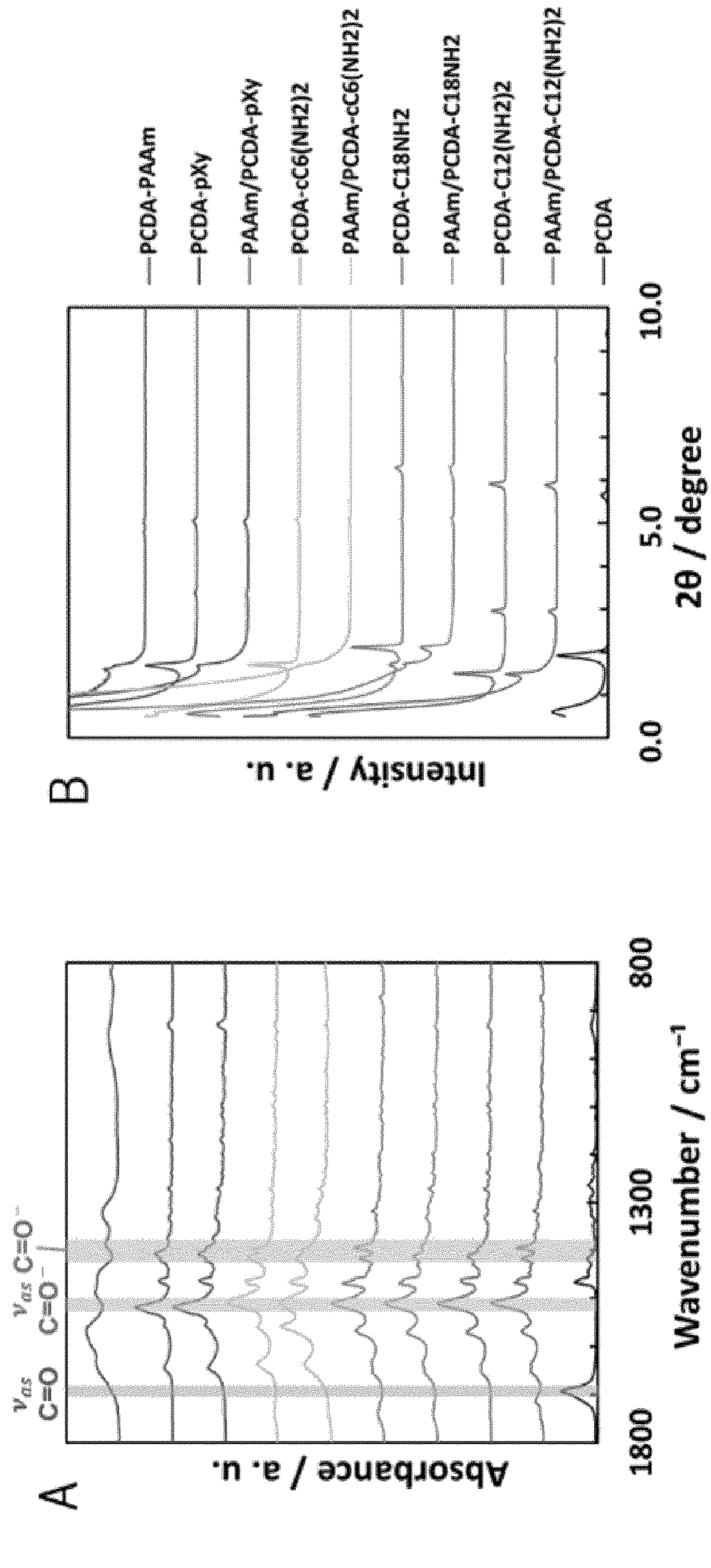
A
Absorbance / a. u.
$\nu_{as}$ C=O
$\nu_{as}$ C=O⁻
C=O⁻
1800
1300
800
Wavenumber / cm⁻¹
B
Intensity / a. u.
0.0
5.0
10.0
2θ / degree
PCDA-PAAm
PCDA-pXy
PAAm/PCDA-pXy
PCDA-cC6(NH2)2
PAAm/PCDA-cC6(NH2)2
PCDA-C18NH2
PAAm/PCDA-C18NH2
PCDA-C12(NH2)2
PAAm/PCDA-C12(NH2)2
PCDA

Fig. 27

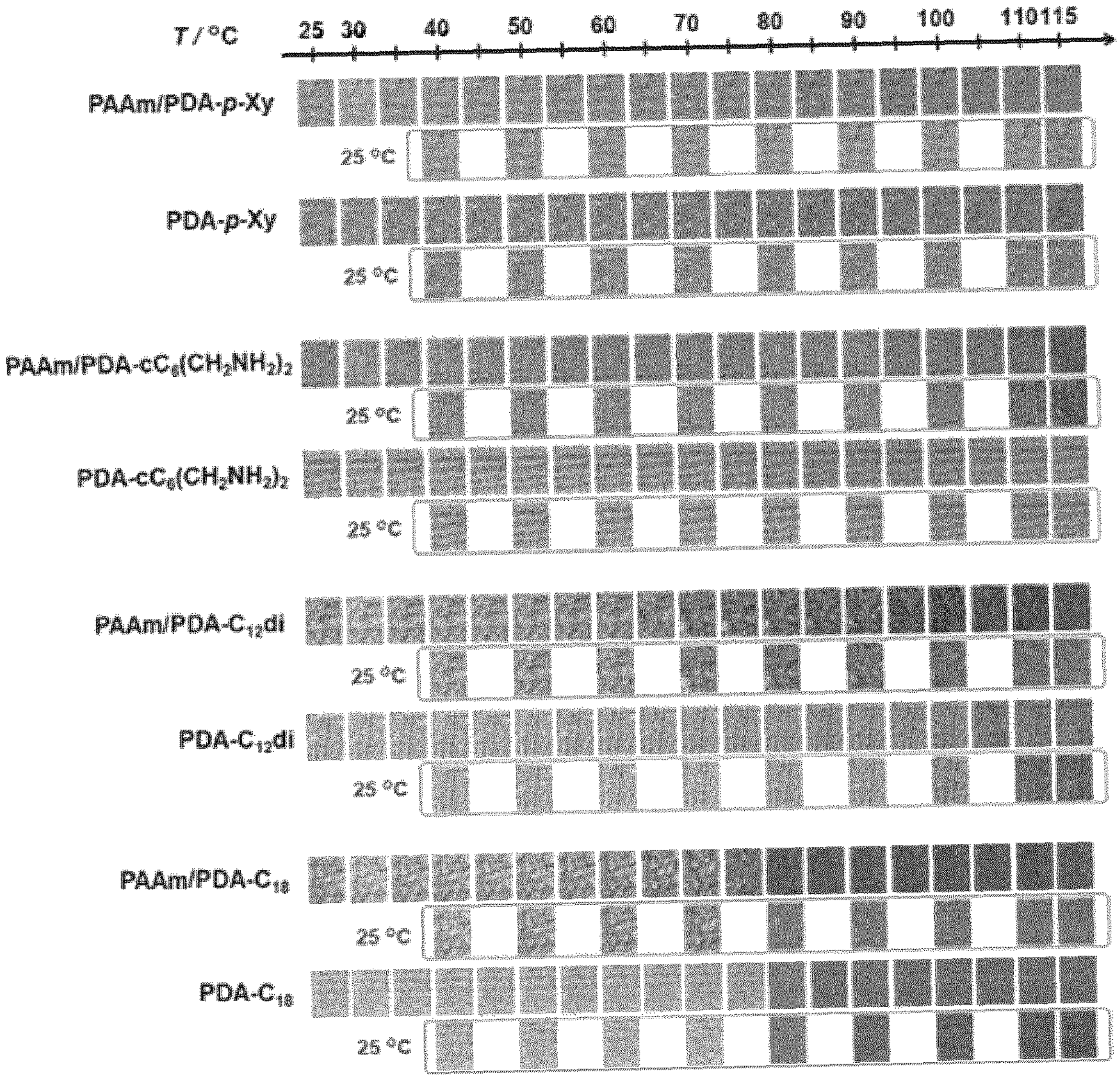
T / °C
25
30
40
50
60
70
80
90
100
110
115
PAAm/PDA-p-Xy
25 °C
PDA-p-Xy
25 °C
PAAm/PDA-cC6(CH2NH2)2
25 °C
PDA-cC6(CH2NH2)2
25 °C
PAAm/PDA-C12di
25 °C
PDA-C12di
25 °C
PAAm/PDA-C18
25 °C
PDA-C18
25 °C

Fig. 28

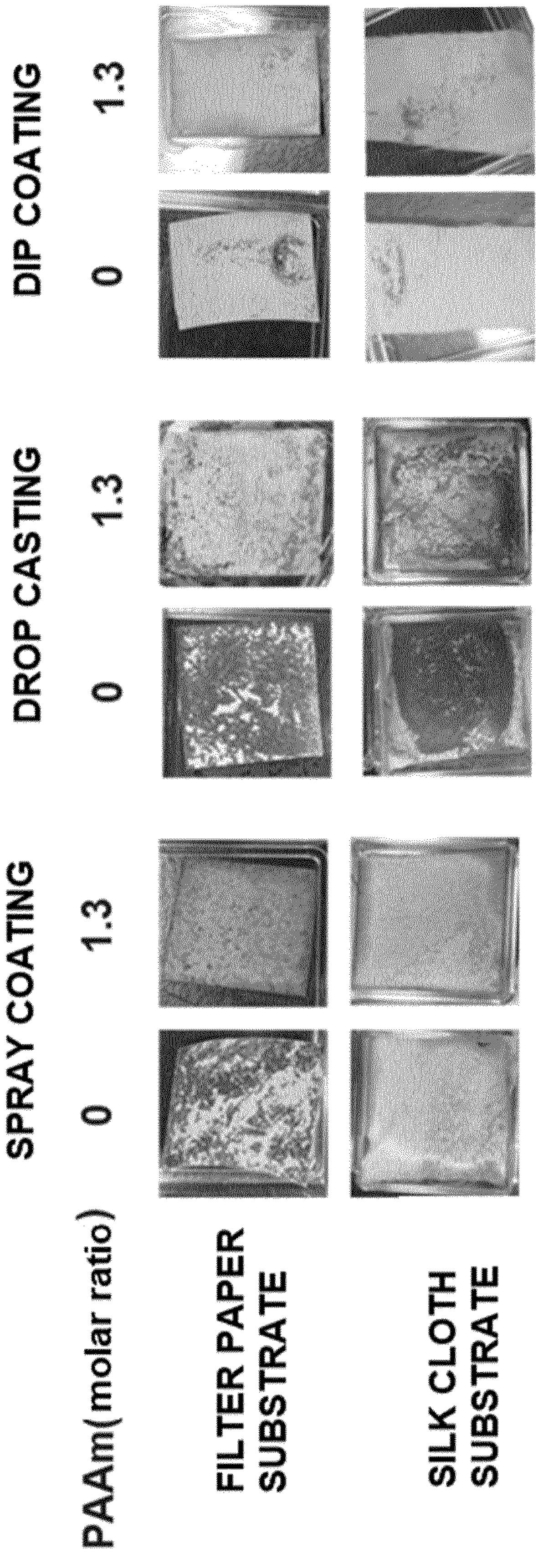
SPRAY COATING
DROP CASTING
DIP COATING
PAAm(molar ratio)
0
1.3
0
1.3
0
1.3
FILTER PAPER SUBSTRATE
SILK CLOTH SUBSTRATE

Fig. 29

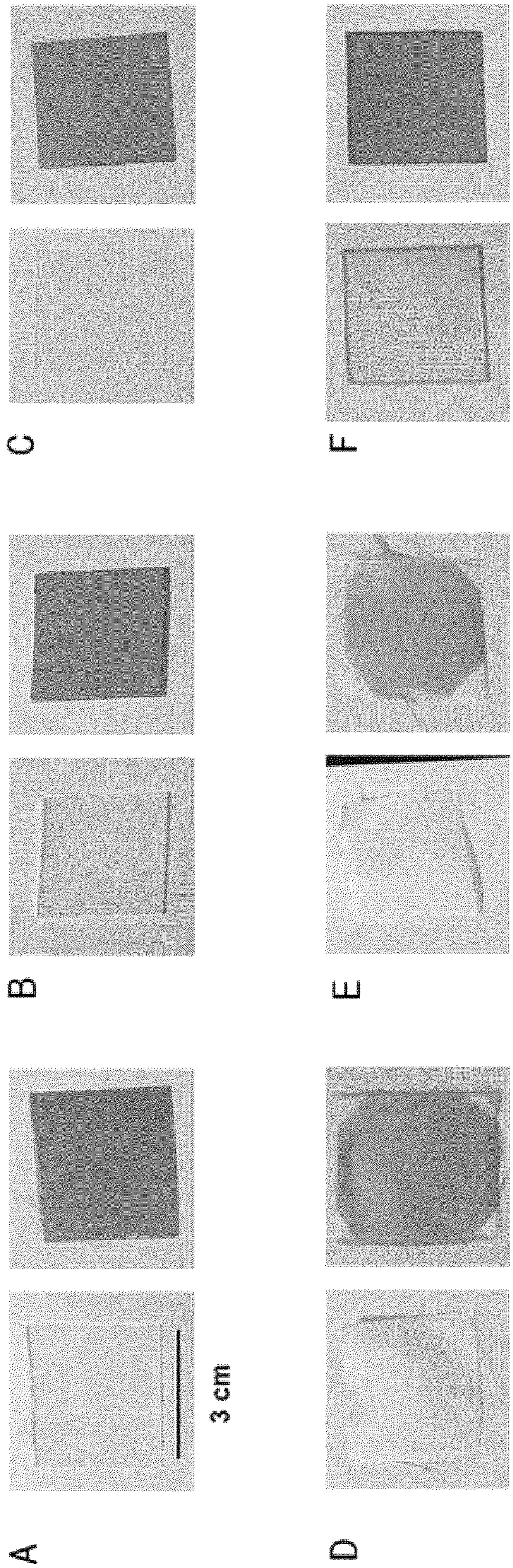

Fig. 30

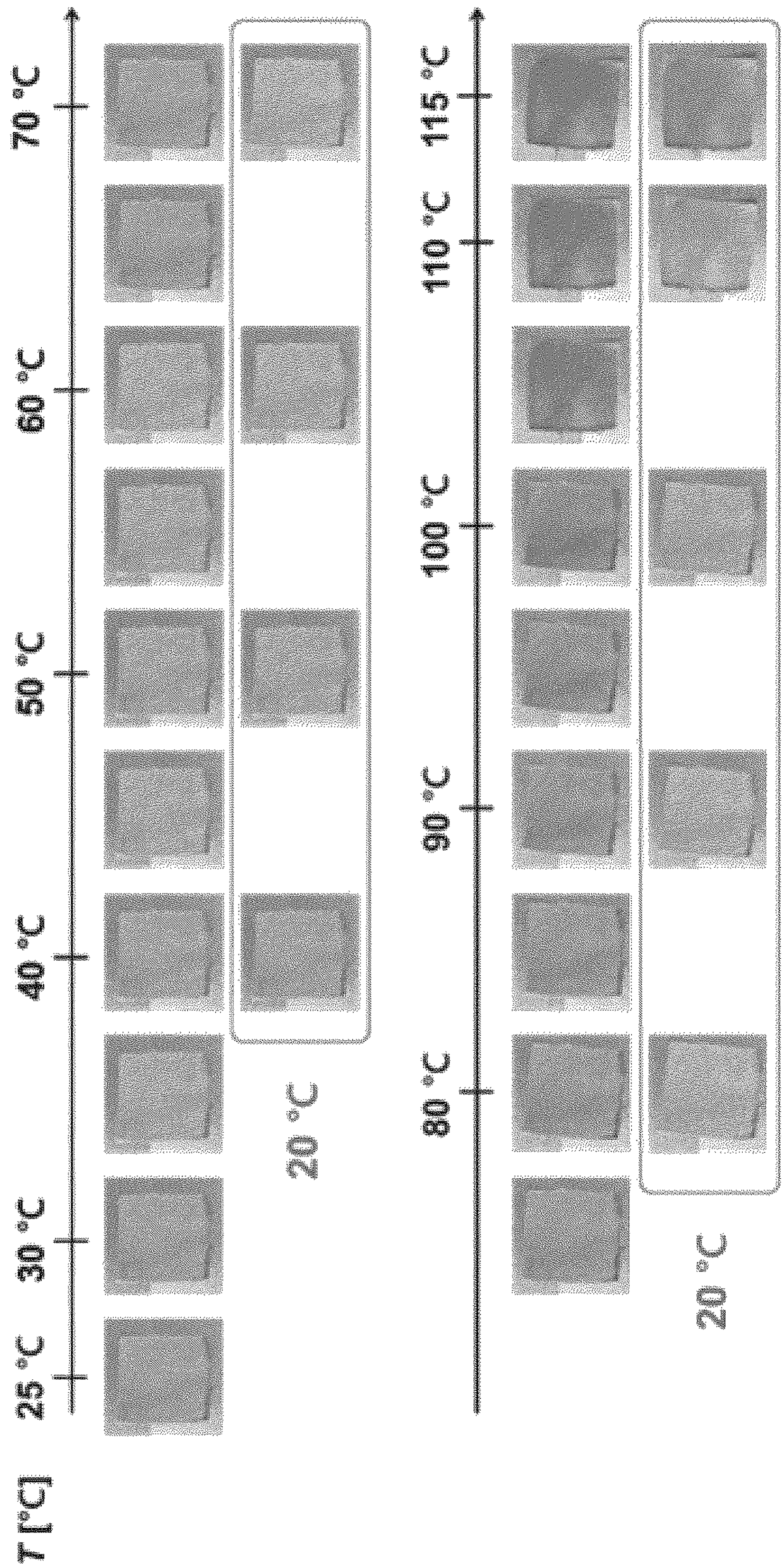

Fig. 31

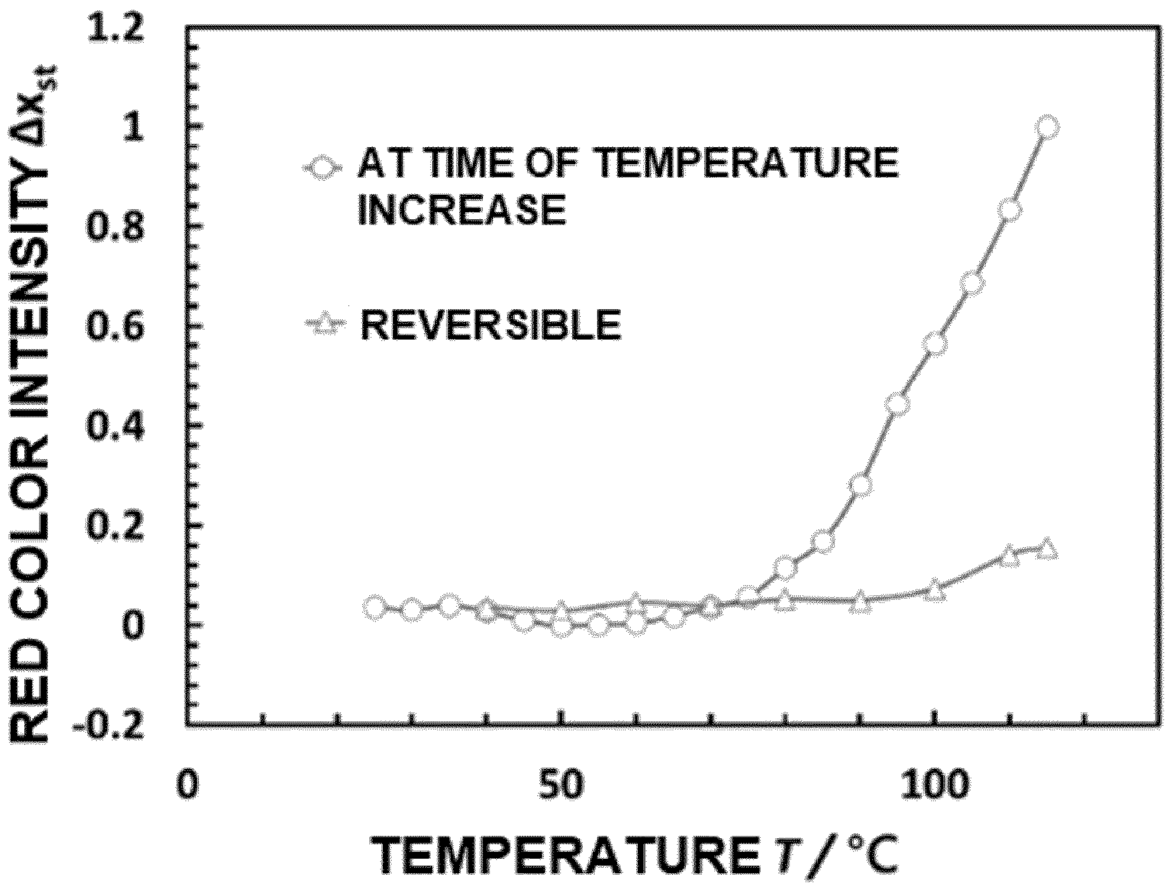
RED COLOR INTENSITY Δx_st
1.2
1
0.8
0.6
0.4
0.2
0
-0.2
AT TIME OF TEMPERATURE INCREASE
REVERSIBLE
0
50
100
TEMPERATURE T / °C

## INTERNATIONAL SEARCH REPORT

International application No.
**PCT/JP2023/016835**

**A. CLASSIFICATION OF SUBJECT MATTER**

***A61B 5/01***(2006.01)i; ***C09K 9/02***(2006.01)i
FI: A61B5/01 100; C09K9/02 C

According to International Patent Classification (IPC) or to both national classification and IPC

**B. FIELDS SEARCHED**

Minimum documentation searched (classification system followed by classification symbols)

A61B1/00-1/32, A61B5/00-5/01, A61B13/00-18/18, A61B34/00-34/20, A61B42/00-90/98, A61F2/01, A61N7/00-7/02

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2021-138793 A (FUTURE COLOR MATERIALS CO., LTD.) 16 September 2021 (2021-09-16) paragraphs [0001]-[0055], fig. 1-3 | 1-16 |
| A | JP 2012-523013 A (DATALASE LTD.) 27 September 2012 (2012-09-27) paragraphs [0001]-[0158] | 1-16 |
| A | WO 2005/012905 A1 (DAIKIN INDUSTRIES, LTD.) 10 February 2005 (2005-02-10) specification, page 1, line 1 to page 13, line 3 | 1-16 |
| A | US 2009/0047488 A1 (CHOI, Yang-Kyu) 19 February 2009 (2009-02-19) paragraphs [0001]-[0065], fig. 1-5 | 1-16 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **30 June 2023** | **11 July 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2023/016835**

| Patent document cited in search report | Publication date (day/month/year) | Patent family member(s) | Publication date (day/month/year) |
|---|---|---|---|
| JP 2021-138793 A | 16 September 2021 | (Family: none) | |
| JP 2012-523013 A | 27 September 2012 | US 2011/0122347 A1<br>paragraphs [0001]-[0106]<br>WO 2010/112940 A1<br>CN 102077140 A<br>KR 10-2011-0046400 A | |
| WO 2005/012905 A1 | 10 February 2005 | US 2007/0042348 A1<br>paragraphs [0001]-[0069]<br>EP 1612543 A1<br>KR 10-2005-0119155 A<br>CN 1768260 A | |
| US 2009/0047488 A1 | 19 February 2009 | KR 10-2009-0016823 A<br><1>-<67>, fig. 1-4 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6801878 B


### Non-patent literature cited in the description


- **M. OKANIWA et al.** *Adv. Funct. Mater.*, 2016, vol. 26, 3463-3471
- **M. TAKEUCHI et al.** *ACS Appl. Mater. Interfaces*, 2017, vol. 9, 16546-16552
- **Y. OAKI** ; **Y. ISHIJIMA** ; **H. IMAI**. *Polymer Journal*, 2018, vol. 50, 319-326
- **AYA EDAGAWA** ; **HIROAKI IMAI** ; **YUYA OAKI**. Reduction in Temperature and Increase in Width of Temperature-responsive Color Change by Flexibility Control of Layered Polydiacetylene. *The 70th SPSJ Annual Meeting*